(19) Europäisches Patentamt / European Patent Office / Office européen des brevets

(11) **EP 4 516 327 A1**

(12) **EUROPEAN PATENT APPLICATION**
published in accordance with Art. 153(4) EPC

(43) Date of publication:
05.03.2025 Bulletin 2025/10

(21) Application number: 23795526.5

(22) Date of filing: 27.04.2023

(51) International Patent Classification (IPC):
*A61L 24/08* (2006.01) *A61L 24/00* (2006.01)
*A61L 31/04* (2006.01) *A61L 31/14* (2006.01)
*A61L 31/16* (2006.01) *A61L 31/18* (2006.01)
*A61K 47/69* (2017.01) *A61K 47/36* (2006.01)
*A61K 45/00* (2006.01)

(86) International application number:
PCT/CN2023/091079

(87) International publication number:
WO 2023/208094 (02.11.2023 Gazette 2023/44)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 28.04.2022 CN 202210478407

(71) Applicant: Zhejiang Sci-Tech University
Hangzhou, Zhejiang 310000 (CN)

(72) Inventors:
• KONG, Xiangdong
Hangzhou, Zhejiang 310000 (CN)
• YI, Zihan
Hangzhou, Zhejiang 310000 (CN)

• ZHAO, Ruibo
Hangzhou, Zhejiang 310000 (CN)
• SUN, Zhichao
Hangzhou, Zhejiang 310000 (CN)
• SHEN, Yang
Hangzhou, Zhejiang 310000 (CN)
• MA, Shitu
Hangzhou, Zhejiang 310000 (CN)
• LUO, Dandan
Hangzhou, Zhejiang 310000 (CN)
• ZU, Boer
Hangzhou, Zhejiang 310000 (CN)

(74) Representative: Bandpay & Greuter
11, rue Christophe Colomb
75008 Paris (FR)

(54) **HOLLOW EMBOLIC MICROSPHERE, PREPARATION METHOD THEREFOR, AND PHARMACEUTICAL COMPOSITION AND USE THEREOF**

(57) A hollow embolic microsphere, a preparation method therefor, and a pharmaceutical composition and use thereof, wherein: the hollow embolic microsphere is a microsphere prepared by using a polymer material and having a unique shape and structure, and is suitable for transcatheter arterial embolization. The hollow embolic microsphere of the present disclosure can enter channels having different sizes, and has good embolization effects on various target positions. The hollow microsphere is internally porous and has drug loading abilities, and drug release when loading drug can be easily controlled and the treatment effect is good. No emulsifier or surfactant is needed in the preparation of the hollow microsphere, reaction conditions are mild, and the process is simple. The obtained microsphere has a uniform shape and a controllable size, and is capable of controlling the embolization time. Various drugs including protein heat-sensitive drugs can be loaded, and the drug encapsulation efficiency can reach more than 70%.

FIG. 1

# EP 4 516 327 A1

**Description**

**Technical Field**

The present disclosure relates to a hollow embolic microsphere, a preparation method therefor, a pharmaceutical composition and use thereof.

**Background**

Transcatheter arterial embolization (TAE) is a widely accepted endovascular treatment, by means of which almost any organ can be reached through vessels, and embolic material can be placed at a target location within the vessel via microcatheters so as to reduce or block blood flow, thereby achieving therapeutic effects. At present, clinical symptoms treated by transcatheter arterial embolization (TAE) mainly include hemorrhagic lesions, arteriovenous malformations, uterine myoma (also known as UAE), prostate hyperplasia (also known as PAE), scar pregnancy, chronic osteoarthritis, uterine adenomyosis, hypersplenism, aneurysms and some tumors (Sheth R A, Sharjeel S, Savitri K. EndovascμLar embolization by transcatheter delivery of particles: past, present, and future. Journal of Functional Biomaterials, 2017, 8(2): 12-20). The quality of embolic agent can greatly affect the therapeutic effects, and is a key factor for successful treatment of patients by means of transcatheter arterial embolization. Metal coil, anhydrous ethanol, iodipin, polyvinyl alcohol and other solid or liquid embolic agents have been extensively used in clinical practice. However, these embolic agents often have some shortcomings or deficiencies, such as toxic side effects, difficulties in delivery in interventional therapy, difficulties in localization of focal sites, difficulties in control of drug release when drug loading, and poor therapeutic effects. Following the continuous development of biomaterial field, more and more researchers are trying to apply some polymer materials or natural polymer materials with fine biocompatibility to vascular embolization (Poursaid A, Jensen M M, Huo E. Polymeric materials for embolic and chemoembolic applications[J]. Journal of Controlled Release, 2016, 240: 414-433).

In recent years, embolic microspheres have attracted extensive attention due to their advantages of easy transportation through catheters and minimally invasive surgery, and have become the most widely used embolic agents among commercial products in clinical practice. Existing embolic microspheres include polyvinyl alcohol microspheres, polylactic acid microspheres, chitosan microspheres, gelatin microspheres, sodium alginate microspheres, etc. (Weng L, Le H C, Talaie R. Bioresorbable hydrogel microspheres for transcatheter embolization: preparation and in vitro evaluation [J]. Journal of VascμLar&Interventional Radiology, 2011, 22(10): 1464-1470.). Most of these embolic microspheres are polymer materials that are degradable in vivo, however, they are all solid microspheres with a certain degree of crosslinking, so it takes at least several months for these embolic microspheres to be completely degraded in vivo, and tends to result in severe hypoxia and cause serious inflammatory reactions, therefore, the therapeutic effects of using these microspheres need to be further improved. For achieving the best embolization treatment effect, it is still goals for scientists to prepare microspheres with more uses, such as microspheres that can load special therapeutic drugs, microspheres that can mark locations of vessels in vivo, microspheres with controllable embolization time, microspheres with high drug loading capacity, and microspheres with high drug encapsulation efficiency.

Currently, the main preparation method of polymer embolic microspheres is the emulsion crosslinking method, in which rather toxic emulsifiers and surfactants are used. The residual amount of these solvents in solid microspheres is large, and it is difficult to completely remove the residues that may lower the biocompatibility of embolic materials and cause unpredictable toxic side effects to human bodies. At the same time, existing embolic microspheres with over-large sizes cannot enter small channels such as small blood vessels, and existing embolic microspheres with over-small sizes cannot embolize large channels such as branch arteries with high blood flow velocity and may be easily flushed to a non-target location by blood flow. In addition, commonly-used preparation methods further include the reversed-phase emulsion synthesis method and the spray drying method, wherein: the reversed-phase emulsion synthesis method will introduce emulsifiers, surfactants and oil-like impurities; and the spray drying method will easily inactivate protein and factor drugs because the molecular structure of drugs may be destroyed by high temperature and the types of carried drugs are limited, moreover, the particle size and morphology of embolic materials prepared by the spray drying method vary greatly, so the prepared microspheres still have many shortcomings even they are screened by means of special screening.

**Summary**

The present disclosure provides a hollow embolic microsphere, a preparation method therefor, a pharmaceutical composition and use thereof.

As for one aspect, the present disclosure provides a hollow embolic microsphere, wherein: the hollow embolic microsphere has a spheroidal or hemispheroidal shape, an internal cavity and an opening structure, and the material of the hollow embolic microsphere is biocompatible polymer material with viscoelasticity; preferably, the hollow embolic microsphere is in a bowl shape, a spherical shape, a hemispherical shape, an ellipsoidal shape, or a short cylinder shape with a spherical head; preferably, a particle diameter range of the hollow embolic microsphere is 50 to 2,000 $\mu$m, preferably 200 to 2,000 $\mu$m, and more preferably 200 to 1,200 $\mu$m; preferably, the hollow embolic microspheres may be a combination of multiple groups of microspheres with different particle size distributions, for example, one or a combination of two or more groups of microspheres with particle size of 50 to 120 $\mu$m, microspheres with particle size of 300 to 500 $\mu$m, microspheres with particle size of 500 to 700 $\mu$m, microspheres with particle size of 700 to 900 $\mu$m, and microspheres with particle size of 900 to 1,200 $\mu$m; for another example, one or a combination of two or more groups of microspheres with particle size of 200 to 300 $\mu$m, 300 to 500 $\mu$m, 500 to 700 $\mu$m, 700 to 900 $\mu$m, and 900 to 1,200 $\mu$m.

Preferably, the inner diameter of the opening structure is 1% to 95% of the particle size of the hollow embolic microsphere; and more preferably, the inner diameter of the opening structure is 10% to 80% of the particle size of the hollow embolic microsphere.

Preferably, the opening of the hollow embolic microsphere has a bulge towards the center of the opening structure or the center of the cavity.

Preferably, if the direction from the top to the opening at the bottom is the height direction, and the direction perpendicular to the height direction is the width direction, then the ratio of width to height of the hollow embolic microsphere is 1:10 to 10:1, preferably 1:3 to 3:1, and more preferably 1:2 to 2:1; the ratio of the inner diameter to the outer diameter of the opening at the opening structure of the hollow embolic microsphere is 1:50 to 9:10, preferably 1:5 to 4:5, and more preferably 1:4 to 1:2; and the ratio of the wall thickness of the hollow embolic microsphere to the microsphere particle size is 1:10 to 1:1, preferably 1:5 to 4:5, and more preferably 1:4 to 2:3.

Preferably, the material of the hollow embolic microsphere is one or more polymers selected from polyvinyl alcohols, polyesters, proteins and sugars; preferably, it is one or more polymers selected from proteins and sugars, more preferably, one or more polymers selected from sugars, and, more preferably, one or more polymers selected from gelatin, alginic acid, chitosan, carboxymethyl cellulose, and hyaluronic acid; more preferably, it is one or more polymers selected from alginic acid, chitosan, carboxymethyl cellulose, hyaluronic acid, and their salts (preferably sodium salt, potassium salt); more preferably, it is one or more polymers selected from sodium alginate, potassium alginate, sodium carboxymethyl cellulose, potassium carboxymethyl cellulose, sodium hyaluronate, potassium hyaluronate, chitosan, carboxymethyl cellulose, and hyaluronic acid; preferably, the relative molecular mass of the hyaluronic acid or hyaluronate used is 10,000 to 20,000,000 daltons, with preferable molecular weight of 100,000 to 8,000,000 daltons, and more preferable molecular weight of 500,000 to 3,000,000 daltons; preferably, the molecular weight of alginate or its salt is 10,000 to 500,000 daltons, and more preferably 100,000 to 200,000 daltons; preferably, the molecular weight of carboxymethyl cellulose or its salt is 10,000 to 1,000,000 daltons, and more preferably 300,000 to 800,000 daltons; preferably, the molecular weight of chitosan is 10,000 to 800,000 daltons, and more preferably 50,000 to 300,000 daltons; preferably, the polymer material is a crosslinked polymer material; preferably, the crosslinking agent used in the crosslinked polymer material is selected from one or a combination of several selected from divinyl sulfone, carbodiimide, dihydrazide adipate, 1,4-butanediol glycidyl ether and glutaraldehyde; more preferably, the crosslinking agent is dihydrazide adipate, 1,4-butanediol glycidyl ether or a combination thereof with other crosslinking agents, and, more preferably is 1,4-butanediol glycidyl ether or a combination thereof with other crosslinking agents; preferably, the mass ratio of the crosslinking agent to the polymer material is 0.0001:1 to 10:1, more preferably 0.01:1 to 2:1, more preferably 0.01:1 to 1:1, more preferably 0.1:1 to 2:1, more preferably 0.1:1 to 1:1.

As for another aspect, the present disclosure provides a method for preparing the hollow embolic microsphere, which is obtained by means of 3D printing.

As for another aspect, the present disclosure provides a method for preparing the hollow embolic microsphere, including steps of:

1) dissolving the polymer material in water, an acidic aqueous solution or an alkaline aqueous solution, and stirring for full dissolution; optionally, adding a crosslinking agent and performing sufficient mixing;
2) adding dropwise the mixed liquid prepared in Step 1) to a dehydrant that is being stirred, and microspheres are formed after dehydration; and
3) collecting the prepared microspheres and drying them;

wherein: there is no restriction on the order for adding the polymer materials and the crosslinking agent in Step 1).

Preferably, as for the above-mentioned preparation method, the concentration of the polymer material prepared in Step 1) is 0.5% to 5%, and preferably 1% to 3.5%; in a case where the polymer material in Step 1) is one or a combination of several of hyaluronic acid, alginic acid and carboxymethyl cellulose, it is preferable that the polymer material is dissolved in water; in a case where the polymer material in Step 1) is a polymer material containing chitosan, it is preferable that the polymer material is dissolved in an acidic aqueous solution, and the pH of the aqueous solution is preferably 1 to 6.9, more preferably 4 to 6.8; preferably, the acidic aqueous solution is hydrochloric acid solution, sulfuric acid solution, nitric acid solution, or glacial acetic acid solution; preferably, the alkaline aqueous solution is sodium hydroxide, or potassium hydroxide aqueous solution.

Preferably, in the above-mentioned preparation method, the crosslinking agent in Step 1) is one or a combination of several selected from divinyl sulfone, carbodiimide, dihydrazide adipate, 1,4-butanediol glycidyl ether, and glutaraldehyde; preferably, the crosslinking agent is dihydrazide adipate, 1,4-butanediol glycidyl ether or a combination thereof with other crosslinking agents, and, more preferably, 1,4-butanediol glycidyl ether or a combination thereof with other crosslinking agents; preferably, the mass ratio of the crosslinking agent to the polymer material is 0.0001:1 to 10:1, more preferably 0.01:1 to 2:1, more preferably 0.01:1 to 1:1, more preferably 0.1:1 to 2:1, and more preferably 0.1:1 to 1:1.

Preferably, in the above-mentioned preparation method, a syringe or microfluidic device is adopted to control the size of the droplets added in the dropwise adding of the mixed liquid in Step 2), so as to obtain desired particle size or a combination of particle size distributions.

Preferably, in the above-mentioned preparation method, the dehydrant in Step 2) is alcohol solvent, preferably alcohol solvent with 3 to 5 carbon atoms, preferably one or several combined solvents with a total content of 95% or more and selected from isopropyl alcohol, isobutyl alcohol, n-amyl alcohol, and isoamyl alcohol, and more preferably one or several combined solvents selected from anhydrous isopropyl alcohol, anhydrous isobutyl alcohol, anhydrous n-amyl alcohol, and anhydrous isoamyl alcohol.

Preferably, in the above-mentioned preparation method, the volume ratio of the dehydrant in Step 2) to the polymer solution is 4:1 or more, preferably 10:1 or more, and more preferably 20:1 or more.

Preferably, in the above-mentioned preparation method, the stirring speed in Step 2) is 100 to 2,000 rpm, and preferably 800 to 1,500 rpm; the dehydration stirring time is 0.1 hours or more, and preferably 0.4 to 4 hours; preferably, 20-50°C oven drying or natural wind evaporation drying or low temperature freeze-drying is adopted in Step 3).

Preferably, the above-mentioned hollow embolic microspheres are drug-loading hollow embolic microspheres; preferably, the loaded drug is one or a combination of several selected from nanoparticles, micron particles, small molecule drugs, chemotherapeutic drugs, fluorescent markers, macromolecular drugs, peptides, plasmids, and viral drugs; preferably, the micron particles or nanoparticles may be listed, for example, as one or a combination of several selected from: metal micron particles, metal nanoparticles, magnetic micron particles, magnetic nanoparticles, inorganic non-metallic micron particles, inorganic non-metallic nanoparticles, polymer micron particles, polymer nanoparticles, nano-micelles, and nano-liposomes; preferably one or a combination of several selected from nano-hydroxyapatite, nano-tantalum powder, nano-calcium phosphate, nano-manganese phosphate, nano-manganese dioxide, nano-iron oxide, nano-barium sulfate, and iohexol; the micron particles can preferably be listed, for example, as one or a combination of several selected from barium sulfate, calcium carbonate microspheres, calcium phosphate microspheres, and aluminum oxide microspheres; the chemotherapeutic drugs, small molecule drugs and macromolecular drugs can be listed, for example, as one a combination of several selected from doxorubicin hydrochloride, paclitaxel, cisplatin, fluorouracil, interferon, aptamers, Sorafenib, Osimertinib, Afatinib, Ceritinib, Palbocillin, Everolimus, Regorafenib, imatinib, Axitinib, Pazopanib, Ibrutinib, Vermotinib, Sonidegib, hyaluronidase, nivolumab, Pertuzumab, Pembrolizumab, Bevacizumab, and antibody conjugated drugs; the fluorescent markers may be listed, for example, as FITC and/or Rhodamine B; the peptides drug may be listed, for example, as one or a combination of several selected from leuprorelin, goserelin, triptorelin, and Nafarelin; the plasmid drugs can be listed, for example, as one or a combination of several selected from pEGFP plasmid, pCMVp-NEO-BAN plasmid, pSV2 plasmid, CMV4 plasmid, pUC18 plasmid, pUC19 plasmid, and pcDNA3.4 plasmid; it is optional that the plasmid drugs carry therapeutic genes, and, preferably, the therapeutic genes include p53 gene, thymidine kinase gene, cytidine deaminase gene, granulo-macrophage colony-stimulating factor gene (GM-CSF), CRISP/Cas9 gene system, anticancer embryo gene, anti-angiogenesis gene, anti-transferrin gene or gene analogitics thereof; and the viral drugs may be listed, for example, as one or a combination of several selected from oncolytic adenoviruses with or without inserted therapeutic genes, oncolytic gland-associated viruses, oncolytic lentiviruses, oncolytic vaccinia viruses, oncolytic herpes simplex viruses, measles viruses, and virus-like microspheres.

Preferably, the preparation method of the drug-loading hollow embolic microsphere includes adding the drugs in Step 1) of the above-mentioned preparation method, wherein: there is no restriction on the order for adding the polymer material, the crosslinking agent and the drug;

alternatively, in the above-mentioned method, after dry microspheres are obtained in Step 3), the microspheres are placed in the drug solution to adsorb the drug; preferably, the microspheres are dried after adsorbing the drug.

Preferably, in Step 1), the mass ratio of the added drug to the polymer material is 0.000001:1 to 10:1, and the mass of the nanoparticles and micron particles added to the polymer material solution per gram of the polymer material is preferably 0.001 to 10 g/g, more preferably 0.01 to 5 g/g; the mass of protein drugs, small molecule drugs, chemotherapeutic drugs and nano drugs added to per gram of polymer materials is at mg level, such as 1 to 2,000 mg/g; the mass of plasmid drugs loaded in each gram of polymer material is at $\mu$g level, such as 1 to 3,000 $\mu$g/g; alternatively, after the microspheres are dried and obtained in Step 3), the microspheres are placed in the drug solution to adsorb drugs, and the mass of protein drugs, small molecule drugs, chemotherapeutic drugs and nano drugs adsorbed by per gram of the polymer material is at mg level, such as 1 to 2,000 mg/g; the mass of plasmid drugs adsorbed by each gram of polymer

material is at $\mu$g level, such as 1 to 3,000 $\mu$g/g; preferably, the dose of viral drugs adsorbed by per cubic centimeter volume of hollow embolic microspheres is 10^6 to 10^12 Pfu/ml (Pfu: plaque-forming units), and preferably 10^7 to 10^10 Pfu/ml, such as oncolytic adenovirus of 10^6 to 10^8 Pfu/ml, oncolytic herpes simplex virus of 10^6 to 10^8 Pfu/ml, and poxvirus of 10^7 to 10^9 Pfu/ml.

The present disclosure further provides a hollow embolic microsphere prepared using the above-mentioned preparation method.

As for another aspect, the present disclosure further provides a pharmaceutical composition or a medical device comprising the above-mentioned hollow embolic microsphere.

As for another aspect, the present disclosure further provides use of the above-mentioned hollow embolic microsphere or the above-mentioned pharmaceutical composition in the preparation of drug or medical device for transcatheter arterial embolization.

As for another aspect, the present disclosure further provides use of the above-mentioned hollow embolic microsphere or pharmaceutical composition in the preparation of drugs for tumors, hemorrhagic lesions, arteriovenous malformations, prostatic hyperplasia (also referred to as PAE), scar pregnancy, chronic osteoarthritis, uterine adenomyosis, and hypersplenism; preferably, the tumors include malignant tumors, or uterine fibroids (also referred to as UAE).

The maximum drug encapsulation efficiency of the drug-loading hollow embolic microspheres can reach 95% or more.

Mass fraction of drug loading capacity can be freely adjusted in a range from 1% to 90%.

**Beneficial Effects**

The present disclosure provides a method for simply and rapidly preparing polysaccharide drug-loading hollow embolic microspheres, wherein the drug-loading hollow embolic microsphere obtained in the present disclosure has a unique hollow structure, and a smooth and mellow outer surface, and existence of a central cavity enables the hollow microsphere to be excellent in viscoelasticity and compressibility after swelling caused by water adsorption, as a result, the hollow embolic microspheres in clinical practice can be easily transported by using microcatheters, and can be easily localized to a lesion site.

Since the drug-loading hollow embolic microsphere has a hollow structure, it can greatly deform in conduits, so as to easily pass through the blood vessel having small diameters, and thus has certain intelligent deformation characteristics and fine embolization effects after arriving at the embolization site. The wall of the microsphere is internally porous, and the pore diameter distribution range is wide, and thus has the performance of easy drug-loading; besides, the drug release can be easily controlled with the drug-loading microsphere, as a result of which the therapeutic effect is fine. The technology of the present disclosure for preparing the drug-loading hollow embolic microspheres is simple, convenient and efficient, and the technology of the present disclosure can be used to quickly prepare drug-loading microspheres of special therapeutic uses, microspheres that can label vessel positions in vivo, and microspheres having controllable embolization time, or the like; the microspheres prepared using the present process have high drug loading capacity and high drug encapsulation efficiency, for instance, the highest drug loading of some drugs is up to 90% and the drug encapsulation efficiency reaches as high as 95% and more, and the drug loading capacity of the microsphere has a large controllable range while the drug encapsulation efficiency is high.

Based on solvent volatilization, the method of the present disclosure adopts cross-linking agent and dehydrant to generate microspheres by means of dehydrate and cross-linking in one step. Compared with reported existing microsphere preparation process, the process of the present disclosure does not introduce emulsifiers, surfactants or other substances in the reaction process, has mild reaction conditions, can load many kinds of drugs and can load multiple drugs at the same time, for instance, it can load protein heat-sensitive drugs, can load nano-hydroxyapatite, nano-tantalum powder, nano drugs and other nanoparticles, can load microparticles such as ultrafine barium sulfate, calcium carbonate microspheres, can load chemotherapeutic drugs, small molecule drugs, macromolecular drugs such as doxorubicin hydrochloride, interferon, targeted drugs, hyaluronidase, antibodies, and can load fluorescent markers such as FITC and Rhodamine B. The drug loading capacity of the microsphere can reach up to 90%, and the drug encapsulation efficiency can reach as high as 95%. By means of the microspheres, one or more drugs can be carried together, it is expectable to exert the synergistic effect of drugs on disease treatment, and the potential clinical application is wide, so the drug-loading hollow embolic microsphere of the present disclosure has a wide clinical application prospect in the field of drug delivery and controllable release. Meanwhile, the microsphere prepared in the present disclosure has uniform particle diameter, controllable size, and mono-dispersion; moreover, the preparation technology is simple and the cost is low. At the same time, the polymer material used for preparing microspheres has high biocompatibility and biosafety, and has been widely approved by FDA, NMPA for clinical applications. A series of animal experiments carried out for the microsphere of the present disclosure further proves that the microsphere has fine vascular embolization effect and X-ray development effect, and is excellent in biocompatibility, biosafety and other characteristics.

**Brief Description of Drawings**

FIG. 1 shows 3D diagrams of a hollow bowl-shaped microsphere;

FIG. 2 shows SEM pictures of the hollow bowl-shaped microspheres prepared in Example 1;

FIG.3 shows Fourier infrared spectrogram of the hollow bowl-shaped microspheres prepared in Example 1;

FIG. 4 shows thermogravimetric curve of the hollow bowl-shaped microspheres prepared in Example 1;

FIG.5. shows Rheological modulus curves of the hollow bowl-shaped microspheres prepared in Example 1, wherein (a) is the energy storage modulus curve and (b) is the loss modulus curve;

FIG. 6 shows SEM picture of hollow bowl-shaped microspheres prepared in Example 2;

FIG. 7 shows Fourier infrared spectrogram, wherein (a) is the Fourier infrared spectrum of the hollow bowl-shaped microspheres in Example 1 with no crosslinking agent added, (b) is the Fourier infrared spectrogram of a crosslinking agent, and (c) is the Fourier infrared spectrum of the hollow bowl-shaped microspheres prepared in Example 2 with crosslinking agents added;

FIG. 8 shows thermogravimetric curves of the hollow bowl-shaped microspheres, wherein (a) is the thermogravimetric curve of the hollow bowl-shaped microspheres in Example 1 with no crosslinking agent added, and (b) is the thermogravimetric curve of the hollow bowl-shaped microspheres prepared in Example 2 with crosslinking agents added;

FIG. 9 shows Rheological modulus curves of the hollow bowl-shaped microspheres prepared in Example 2, wherein (a) is the energy storage modulus curve and (b) is the loss modulus curve;

FIG. 10 shows size distribution diagram of the hollow bowl-shaped microspheres prepared with respectively needles of 32 G, 30 G and 28 G in Example 2;

FIG. 11 shows stereoscopic pictures of the hollow bowl-shaped microspheres prepared with respectively needles of 32 G, 30 G and 28 G in Example 2;

FIG. 12 shows stereoscopic pictures of the hollow bowl-shaped microspheres prepared in Example 3;

FIG. 13 shows SEM pictures of the hollow bowl-shaped microspheres prepared in Example 3;

FIG. 14 shows Fourier infrared spectrum of the hollow bowl-shaped microspheres prepared in Example 3, wherein (a) is the Fourier infrared spectrum of the sodium alginate powder, and (b) is the Fourier infrared spectrum of the sodium alginate hollow bowl-shaped microspheres prepared in Example 6;

FIG. 15 shows Rheological modulus curves of the hollow bowl-shaped microspheres prepared in Example 3, wherein (a) is the energy storage modulus curve and (b) is the loss modulus curve;

FIG. 16 shows stereoscopic pictures of the hollow bowl-shaped microspheres prepared in Example 4;

FIG. 17 shows SEM pictures of the hollow bowl-shaped microspheres prepared in Example 4;

FIG. 18 shows Fourier infrared spectrum, wherein (a) is the Fourier infrared spectrum of chitosan powder, and (b) is the Fourier infrared spectrum of the chitosan hollow bowl-shaped microspheres prepared in Example 7;

FIG. 19 shows Rheological modulus curves of the hollow bowl-shaped microspheres prepared in Example 4, wherein (a) is the energy storage modulus curve and (b) is the loss modulus curve;

FIG. 20 shows SEM pictures of the hollow bowl-shaped microspheres prepared in Example 5;

FIG. 21 shows Fourier infrared spectrum, wherein (a) is the Fourier infrared spectrum of carboxymethylcellulose powder, and (b) is the Fourier infrared spectrum of the carboxymethylcellulose hollow bowl-shaped microspheres prepared in Example 5;

FIG. 22 shows Rheological modulus curves of the hollow bowl-shaped microspheres prepared in Example 5, wherein (a) is the energy storage modulus curve and (b) is the loss modulus curve.

FIG. 23 shows Fourier infrared spectrum of sodium hyaluronate bowl-shaped microspheres that are loaded with iohexol and prepared in Example 6, wherein (a) is sodium hyaluronate bowl-shaped hollow microspheres, (b) is iohexol powder, and (c) is sodium hyaluronate bowl-shaped hollow microspheres loaded with 50 mg of iohexol.

FIG. 24 shows SEM pictures of the sodium hyaluronate bowl-shaped hollow microspheres that are prepared in Example 6 and loaded with 50 mg of iohexol.

FIG. 25 shows energy spectrum picture of sodium hyaluronate bowl-shaped hollow microspheres that are prepared in Example 6 and loaded with 50 mg of iohexol.

FIG. 26 shows thermogravimetric curves of sodium hyaluronate bowl-shaped hollow microspheres that are prepared in Example 6 and loaded with 50 mg of iohexol.

FIG. 27 shows CT images of the bowl-shaped hollow microsphere prepared in Example 6, wherein: the left image shows the sodium hyaluronate bowl-shaped hollow microsphere; and the right image shows the sodium hyaluronate bowl-shaped hollow microsphere loaded with 50 mg of iohexol.

FIG. 28 shows in vitro drug release by the sodium hyaluronate bowl-shaped hollow microspheres that are prepared in Example 6 and loaded with 50 mg of iohexol.

FIG. 29 shows stereoscopic pictures of sodium hyaluronate microspheres loaded with iohexol of different weights in Example 6, wherein (a) shows sodium hyaluronate loaded with 0.2 g of iohexol, the mass ratio of sodium hyaluronate to iohexol being 1:2, and the scale being 1 cm; (b) shows sodium hyaluronate loaded with 0.5 g of iohexol, the mass ratio of sodium hyaluronate to iohexol being 1:5, and the scale being 1 cm.

FIG. 30 shows SEM pictures of sodium hyaluronate bowl-shaped hollow microspheres that are prepared in Example 7 and loaded with 50 mg of tantalum nanoparticles.

FIG.31 shows energy spectrum picture of sodium hyaluronate bowl-shaped hollow microspheres that are prepared in Example 7 and loaded with 50 mg of tantalum nanoparticles.

FIG. 32 shows thermogravimetric curves of bowl-shaped hollow microspheres prepared in Example 7, wherein (a) is sodium hyaluronate bowl-shaped hollow microspheres, (b) is sodium hyaluronate bowl-shaped hollow microspheres added with 50 mg of tantalum nanoparticles, and (c) is tantalum nanoparticles.

FIG. 33 shows stereoscopic pictures of sodium hyaluronate microspheres loaded with nano-tantalum powder of different weights in Example 7, wherein (a) shows sodium hyaluronate loaded with 1 g of nano-tantalum powder, the mass ratio of sodium hyaluronate to nano-tantalum powder being 1:10, and the scale being 1 cm; (b) shows sodium hyaluronate loaded with 2 g of nano-tantalum powder, the mass ratio of sodium hyaluronate to nano-tantalum powder being 1:20, and the scale being 1 cm.

FIG. 34 shows SEM pictures of sodium hyaluronate bowl-shaped hollow microspheres that are prepared in Example 8 and loaded with 0.4 mg of barium sulfate.

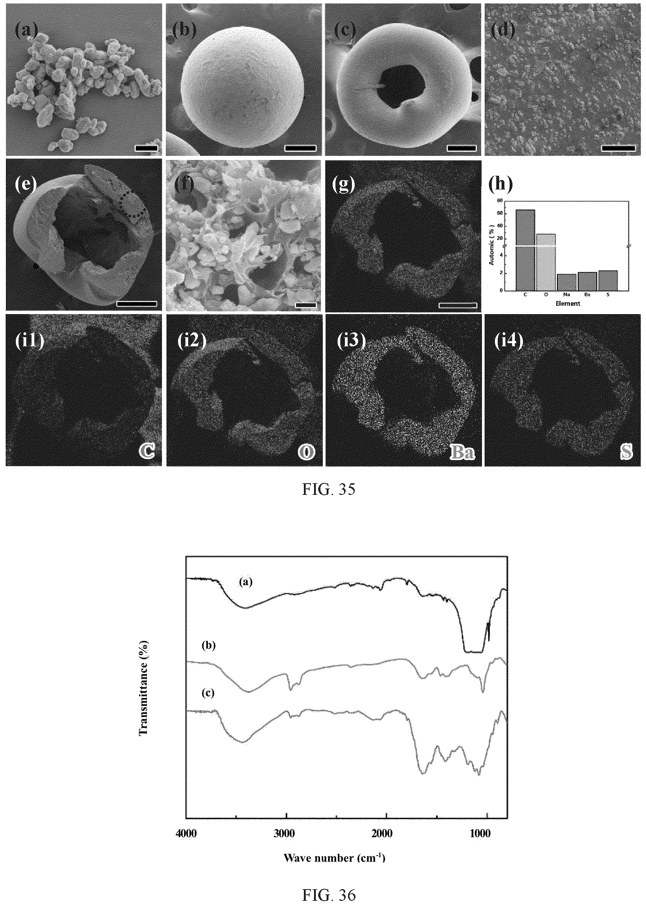

FIG. 35 shows barium sulfate element distribution diagram in sodium hyaluronate bowl-shaped hollow microspheres that are prepared in Example 8 and loaded with 0.4 g of barium sulfate, wherein (a) is the SEM picture of barium sulfate, (b) is the SEM picture of the top of the hollow bowl-shaped embolic microsphere, (c) is the SEM picture of the bottom of the hollow bowl-shaped embolic microsphere, (d) is the partial enlarged SEM picture of the bottom of the hollow bowl-shaped embolic microsphere, (e) is the SEM picture of the cross section of the hollow bowl-shaped microsphere, (f) is the partial enlarged SEM picture of the cross section of the hollow bowl-shaped microsphere, (g) is the cross section element distribution diagram of the hollow bowl-shaped microsphere, and (h) is the bar diagram of content proportions of elements including C, O, Na, Ba and S in the hollow bowl-shaped microsphere; (i1) is the distribution of element C in sodium hyaluronate bowl-shaped microspheres loaded with 0.4 g of barium sulfate, (i2) is the distribution of element O in sodium hyaluronate bowl-shaped microspheres loaded with 0.4 g of barium sulfate, (i3) is the distribution of element Ba in sodium hyaluronate bowl-shaped microspheres loaded with 0.4 g of barium sulfate, and (i4) is the distribution of element S in sodium hyaluronate bowl-shaped microspheres loaded with 0.4 g of barium sulfate.

FIG. 36 shows Fourier infrared spectrum of bowl-shaped hollow microspheres prepared in Example 8, wherein: (a) is barium sulfate, (b) is sodium hyaluronate bowl-shaped hollow microspheres, and (c) is sodium hyaluronate bowl-shaped hollow microspheres loaded with 0.4 g of barium sulfate.

FIG. 37 is diagram of the hollow bowl-shaped microspheres, prepared in Example 8 and loaded with 0.4 g, 0.2 g, 0.1 g, 0.05 g of barium sulfate, compressed in a channel.

FIG. 38 shows thermogravimetric curves of sodium hyaluronate hollow bowl-shaped microspheres that are prepared in Example 8 and loaded with 0.4 g, 0.2 g, 0.1 g, and 0.05 g of barium sulfate.

FIG. 39 shows the energy spectrum picture of sodium hyaluronate bowl-shaped hollow microspheres that are prepared in Example 8 and loaded with 0.4 g of barium sulfate.

FIG. 40 shows CT images of sodium hyaluronate bowl-shaped hollow microspheres that are prepared in Example 8 and loaded with barium sulfate.

FIG. 41 shows in vitro biocompatibility images of sodium hyaluronate bowl-shaped hollow microspheres that are prepared in Example 8 and loaded with 0.4 g of barium sulfate, wherein: (a) is the clotting experiment, (b) is the hemolytic experiment, (c) is a diagram showing results of HUVEC cell proliferation assay measured by means of CCK-8 method, and (d) is a diagram showing growth of HUVEC cells observed by means of live and dead dyeing.

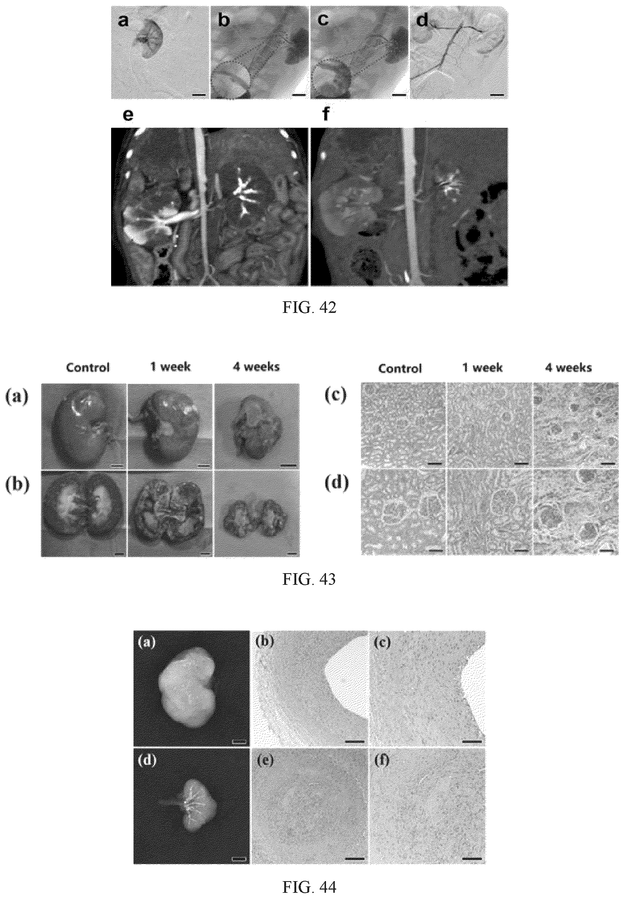

FIG. 42 shows diagrams of beagle kidney embolization by means of sodium hyaluronate bowl-shaped hollow microspheres that are prepared in Example 8 and loaded with 0.4 g of barium sulfate, wherein: (a) shows the image of a normal kidney under DSA without embolization after injection of iohexol solution; (b) and (c) are images of a catheter under DSA, which enters into the body by passing through the renal artery via blood vessels so as to deliver sodium hyaluronate bowl-shaped hollow microspheres loaded with 0.4 g of barium sulfate; (d) shows the image under DSA after embolization of the kidney with microspheres; and (e) and (f) are CT images of the kidney after embolization.

FIG. 43 shows pathological examination of tissue of a beagle kidney embolized by means of sodium hyaluronate bowl-shaped hollow embolic microspheres that are prepared in Example 8 and loaded with 0.4 g of barium sulfate, wherein: (a) shows general images of an embolized kidney after 1 and 4 weeks after surgery and that of a normal kidney, with a scale of 1 cm; (b) shows cross-sectional pictures of (a) with a scale of 1 cm; (c) shows H&E dyeing at the first and fourth week of embolization and that of the normal kidney, with a scale of 100 $\mu$m; and (d) shows enlarged images of (c) with a scale of 200 $\mu$m.

FIG. 44 shows histopathological examination of tissue of a beagle kidney embolized by means of sodium hyaluronate bowl-shaped hollow embolic microspheres that are prepared in Example 8 and loaded with 0.4 g of barium sulfate, wherein: (a) shows the CT image of an unembolized kidney at the fourth week, with a scale of 2 cm; (b) shows H&E dyeing of renal artery in (a), with a scale of 100 $\mu$m; (c) shows an enlarged picture of (b), with a scale of 200 $\mu$m; (d) shows the CT image of the embolized kidney at the fourth week, with a scale of 2 cm; (e) shows H&E dyeing of renal artery in (d), with a scale of 100 $\mu$m; and (f) is an enlarged image of (e), with a scale of 200 $\mu$m.

FIG. 45 shows SEM pictures of cross section of bowl-shaped microspheres that are prepared in Example 11 and loaded with 50 mg of HAp-Fe$_3$O$_4$ nanoparticles.

FIG. 46 shows an energy spectrum picture of sodium hyaluronate bowl-shaped hollow microspheres that are prepared in Example 11 and loaded with 50 mg of HAp-Fe$_3$O$_4$ nanoparticles.

FIG. 47 shows thermogravimetric curves of sodium hyaluronate bowl-shaped hollow microspheres prepared in Example 11, wherein: (a) is sodium hyaluronate bowl-shaped hollow microspheres; (b) is sodium hyaluronate bowl-shaped hollow microspheres that are loaded with 50 mg of HAp-Fe$_3$O$_4$ nanoparticles; and (c) is HAp-Fe$_3$O$_4$ nanoparticles.

FIG. 48 shows SEM pictures of sodium hyaluronate bowl-shaped hollow microspheres that are prepared in Example 12 and loaded with 50 mg of Mn$_3$O$_4$ nanoparticles.

FIG. 49 shows energy spectrum picture of sodium hyaluronate bowl-shaped hollow microspheres that are prepared in Example 12 and loaded with 50 mg of Mn$_3$O$_4$ nanoparticles.

FIG. 50 shows stereoscopic images of sodium hyaluronate microspheres that are prepared in Example 13 and loaded with doxorubicin hydrochloride of different weights, wherein (a) is a case where sodium hyaluronate is loaded with 5 mg of doxorubicin hydrochloride, with a mass ratio of sodium hyaluronate to doxorubicin hydrochloride being 20:1 and the scale being 1 cm; and (b) is a case where sodium hyaluronate is loaded with 8 mg of doxorubicin hydrochloride, with a mass ratio of sodium hyaluronate to doxorubicin hydrochloride being 25:2 and a scale being 1 cm.

FIG. 51 shows Fourier infrared spectrum of bowl-shaped hollow microspheres prepared in Example 13, wherein: (a) represents sodium hyaluronate bowl-shaped hollow microspheres, (b) represents doxorubicin hydrochloride powder, and (c) represents sodium hyaluronate bowl-shaped hollow microspheres loaded with 5 mg of doxorubicin hydrochloride powder.

FIG. 52 shows Fourier infrared spectrum of bowl-shaped hollow microspheres prepared in Example 14, wherein: (a) represents sodium hyaluronate bowl-shaped hollow microspheres, (b) represents paclitaxel powder, and (c) represents sodium hyaluronate bowl-shaped hollow microspheres loaded with 5 mg of paclitaxel powder.

FIG. 53 shows in vitro drug release diagrams of sodium hyaluronate bowl-shaped hollow microspheres that are prepared in Example 15 and respectively loaded with 12 mg of Rhodamine B and doxorubicin hydrochloride.

**Examples**

The present disclosure will be further illustrated hereinafter with reference to the Examples.

**Example 1: Preparation and characterization of sodium hyaluronate hollow bowl-shaped microspheres**

0.4 g of sodium hyaluronate powder, of which the molecular weight is 1 to 1.5 million daltons, was added into 20 ml of hydrochloric acid solution, of which pH is 5, and then was subject to stirring at room temperature for 24 hours at 500 rpm till sufficient dissolution of the sodium hyaluronate powder, thereby obtaining injectable sodium hyaluronate gel. The gel can be stored at room temperature.

By using a syringe of 1 ml, with a needle of 28 G having a beveled opening, the gel was slowly added dropwise into a beaker filled with 40 ml of isobutol, and then was subject to stirring for 60 minutes at 1,200 rpm till the gel droplets in the solution changed from transparent to white solid microspheres.

Obtained solid microspheres were filtered out, and were placed in an oven at 25°C for 48 hours until the mass of the microspheres remained constant, thereby obtaining un-crosslinked sodium hyaluronate hollow microspheres.

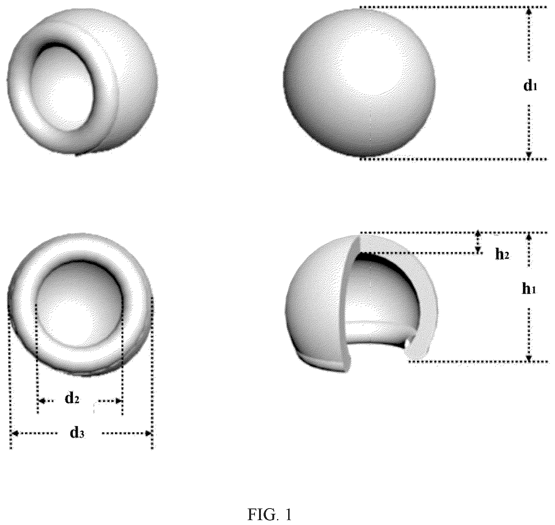

FIG. 1 shows 3D diagrams of hollow bowl-shaped microspheres prepared in Example 1;

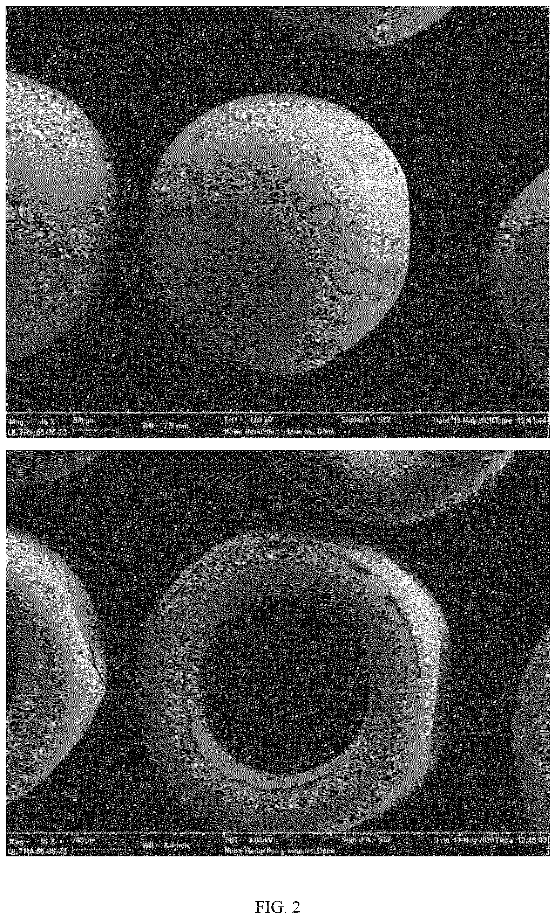

FIG. 2 shows SEM pictures of the hollow bowl-shaped microspheres prepared in Example 1; as shown in the drawing, the microspheres prepared in Example 1 are hemispherical, and have a cavity at interior and a circular opening structure at the round bottom thereof.

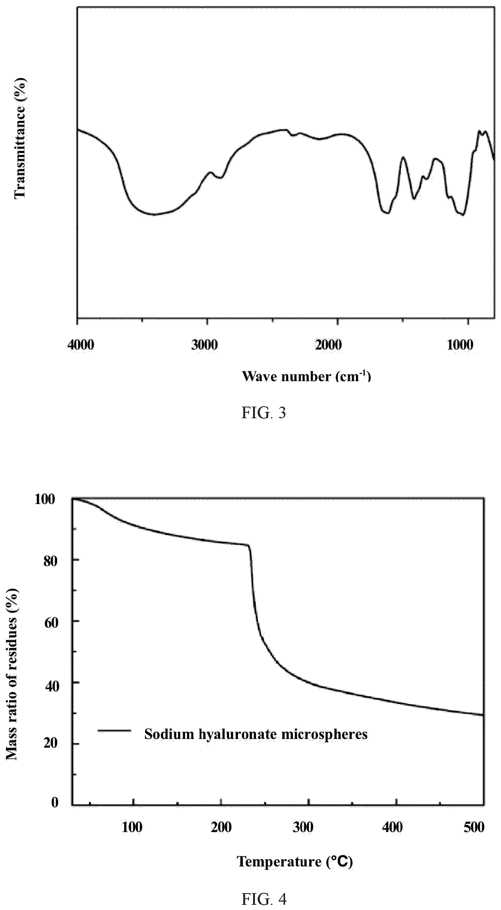

FIG. 3 shows Fourier infrared spectrogram of the hollow bowl-shaped microspheres prepared in Example 1, which has sodium hyaluronate characteristic peaks, indicating that the preparation process is a simple dehydration process without changing the material structure.

FIG. 4 shows thermogravimetric curve of the hollow bowl-shaped microspheres prepared in Example 1, wherein the percentage of weight near 220°C decreases rapidly, indicating significant weight loss around this temperature.

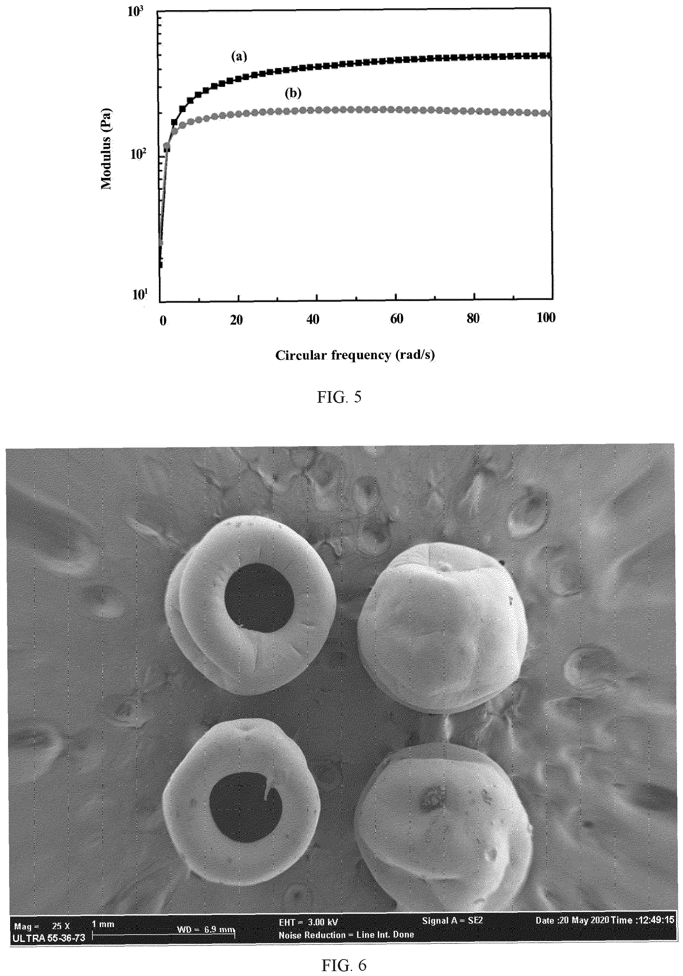

FIG. 5 shows Rheological modulus curves of the hollow bowl-shaped microspheres prepared in Example 1, wherein (a) is the energy storage modulus curve and (b) is the loss modulus curve. As shown in the drawing, the energy storage modulus is always higher than the loss modulus, indicating that the obtained microsphere is an elastic solid with excellent viscoelasticity and the ability of compressive deformation.

**Example 2: Preparation and characterization of sodium hyaluronate hollow bowl-shaped microspheres**

50 ul of 1,4-butanediol glycyl ether was added into 20 ml of hydrochloric acid solution, of which pH is 5, and was subject to stirring for 30 minutes at 500 rpm; 0.4 g of sodium hyaluronate powder, of which the molecular weight is 1 to 1.5 million daltons, was added into the mixed solution, and was subject to stirring at room temperature for 24 hours at 500 rpm till sufficient dissolution of the sodium hyaluronate powder, thereby obtaining sodium hyaluronate gel. The gel can be stored at room temperature.

Using syringes of 1 ml, respectively with needles of 32 G, 30 G, and 28 G having a beveled opening, the gel was slowly added dropwise into a beaker filled with 40 ml of n-amyl alcohol, and then was subject to stirring for 100 minutes at 1,200 rpm till the gel droplets in the solution changed from transparent to white solid microspheres.

The obtained solid microspheres were filtered out, and were placed in an oven at 40°C for 48 hours until the mass of the microspheres remained constant, thereby obtaining sodium hyaluronate hollow microspheres.

FIG. 6 shows SEM picture of the hollow bowl-shaped microspheres prepared in Example 2; as shown in the drawing, the microspheres prepared in Example 2 are hemispherical, and have a cavity at interior and a circular opening structure at the round bottom thereof.

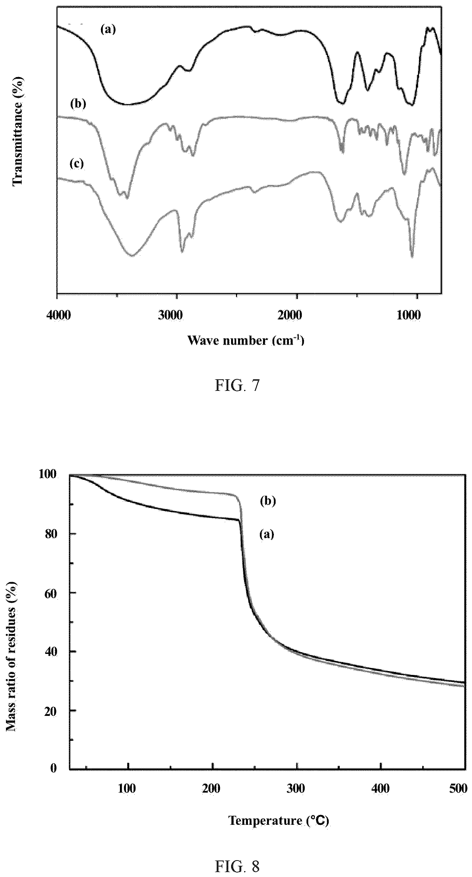

FIG. 7 shows Fourier infrared spectrogram, wherein (a) is Fourier infrared spectrum of the hollow bowl-shaped microspheres in Example 1 with no crosslinking agent added, (b) is Fourier infrared spectrogram of a crosslinking agent, and (c) is Fourier infrared spectrum of the hollow bowl-shaped microspheres prepared in Example 2 with a crosslinking agent added. The diagram proves successful internal crosslinking of microspheres.

FIG. 8 shows thermogravimetric curves of the hollow bowl-shaped microspheres, wherein (a) is the thermogravimetric curve of the hollow bowl-shaped microspheres in Example 1 with no crosslinking agent added, and (b) is the thermogravimetric curve of the hollow bowl-shaped microspheres prepared in Example 2 with a crosslinking agent added; significant differences between these two curves indicate successful internal crosslinking.

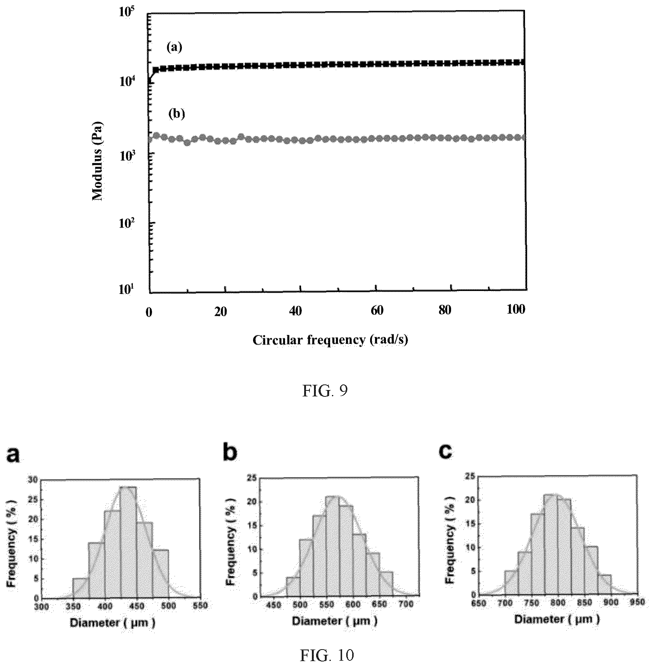

FIG. 9 shows Rheological modulus curves of the hollow bowl-shaped microspheres prepared in Example 2, wherein (a) is the energy storage modulus curve and (b) is the loss modulus curve. As shown in the drawing, the energy storage modulus is higher than the loss modulus, indicating that the obtained microsphere is an elastic solid with excellent viscoelasticity and the ability of compressive deformation.

FIG. 10 shows size distribution diagrams of the hollow bowl-shaped microspheres prepared with respectively needles of 32G, 30G and 28G in Example 2.

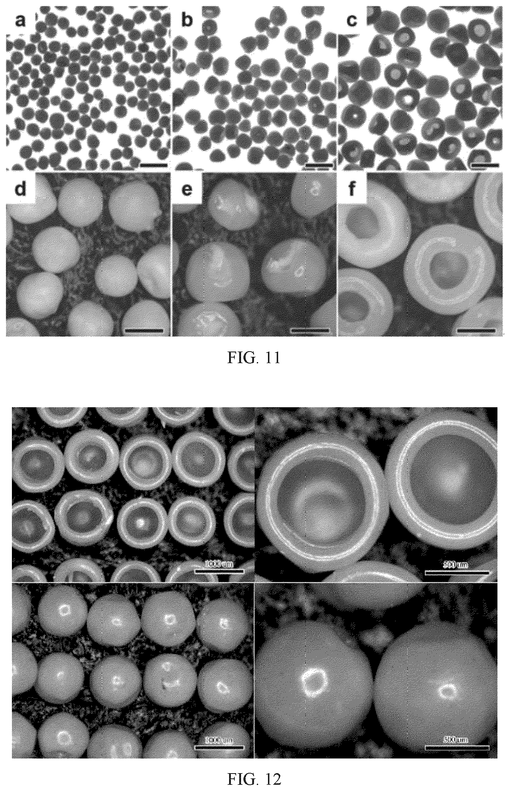

FIG. 11 shows stereoscopic pictures of the hollow bowl-shaped microspheres prepared with respectively needles of 32 G, 30 G and 28 G in Example 2, wherein: (a) shows microspheres prepared using a needle of 32 G, with a scale of 1 mm; (b) shows microspheres prepared using a needle of 30 G, with a scale of 1 mm; (c) shows microspheres prepared using a needle of 28 G, with a scale of 1 mm; and (d-f) are respectively enlarged pictures of (a-c), with a scale of 400 $\mu$m.

**Example 3: Preparation of sodium alginate hollow bowl-shaped microspheres**

400 ul of 1,4-butanediol glycidyl ether was added into 15 ml of hydrochloric acid solution, of which pH is 5, and was subject to stirring for 30 minutes at 500 rpm; 0.3 g of sodium alginate powder, of which the viscosity is 200 to 500 mPa.s, was added into the mixed solution, and was subject to stirring at room temperature for 20 hours at 500 rpm till sufficient dissolution of the sodium alginate powder, thereby obtaining sodium alginate gel. The gel can be stored at room temperature.

By using a syringe of 1 ml with a needle of 28 G having a beveled opening, the gel was slowly added dropwise into a beaker filled with 40 ml of isobutyl alcohol, and then was subject to stirring for 120 minutes at 1000 rpm till the gel droplets in the solution changed from semitransparent yellow to faint yellow solid microspheres.

The obtained solid microspheres were filtered out, and were placed in an oven at 40°C for 12 hours until the mass of the microspheres remained constant.

FIG. 12 shows stereoscopic pictures of the hollow bowl-shaped microspheres prepared in Example 3.

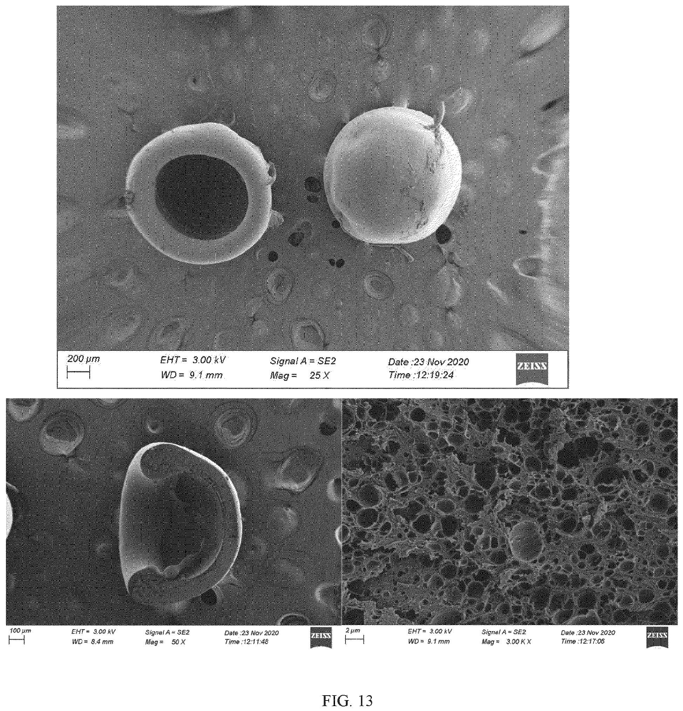

FIG. 13 shows SEM pictures of the hollow bowl-shaped microspheres prepared in Example 3; as shown in the drawing, the prepared microsphere is hemispherical, and has a cavity at interior and a circular opening structure at the round bottom thereof.

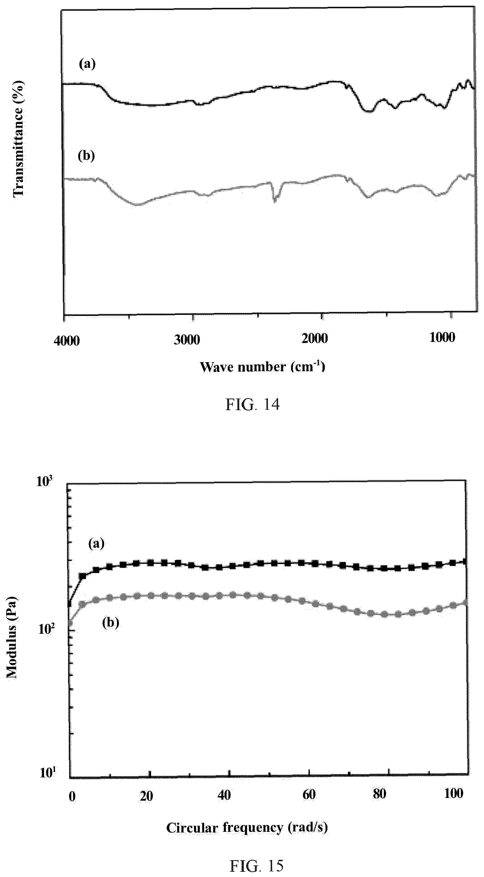

FIG. 14 shows Fourier infrared spectrum, wherein (a) is the Fourier infrared spectrum of the sodium alginate powder, and (b) is the Fourier infrared spectrum of the sodium alginate hollow bowl-shaped microspheres prepared in Example 3; the comparison of these two curves indicates successful internal crosslinking of microspheres.

FIG. 15 shows Rheological modulus curves of the hollow bowl-shaped microspheres prepared in Example 3, wherein (a) is the energy storage modulus curve and (b) is the loss modulus curve. As shown in the drawing, the energy storage modulus is higher than the loss modulus, indicating that the obtained microsphere is an elastic solid with excellent viscoelasticity and the ability of compressive deformation.

**Example 4: Preparation of chitosan hollow bowl-shaped microspheres**

400 ul of 1,4-butanediol glycidyl ether was added into 20 ml of glacial acetic acid solution, of which the mass fraction is 2%, and was subject to stirring for 30 minutes at 500 rpm; 0.6 g of chitosan powder, of which the viscosity is 100 to 200 mPa.s and the deacetylation degree is 95% or higher, was added into the mixed solution, and was subject to stirring at room temperature for 24 hours at 800 rpm till sufficient dissolution of the chitosan powder, thereby obtaining chitosan gel. The gel can be stored at room temperature.

By using a syringe of 1 ml with a needle of 28 G having a beveled opening, the gel was slowly added dropwise into a beaker filled with 40 ml of isoamyl alcohol, and then was subject to stirring for 180 minutes at 600 rpm till the gel droplets in the solution changed from semitransparent to milk-white solid microspheres.

The obtained solid microspheres were filtered out, and were placed in an oven at 60°C for 6 hours until the mass of the microspheres remained constant.

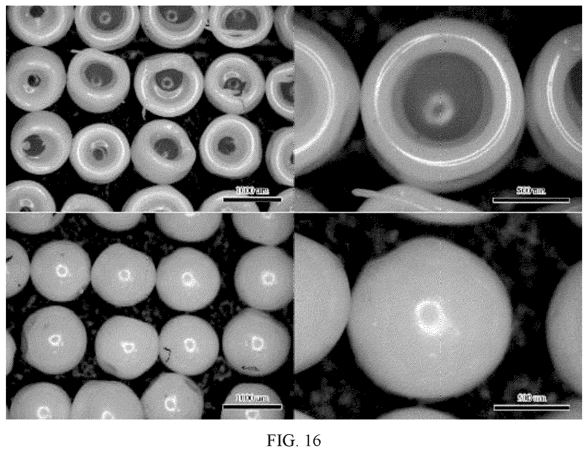

FIG. 16 shows stereoscopic pictures of the hollow bowl-shaped microspheres prepared in Example 4.

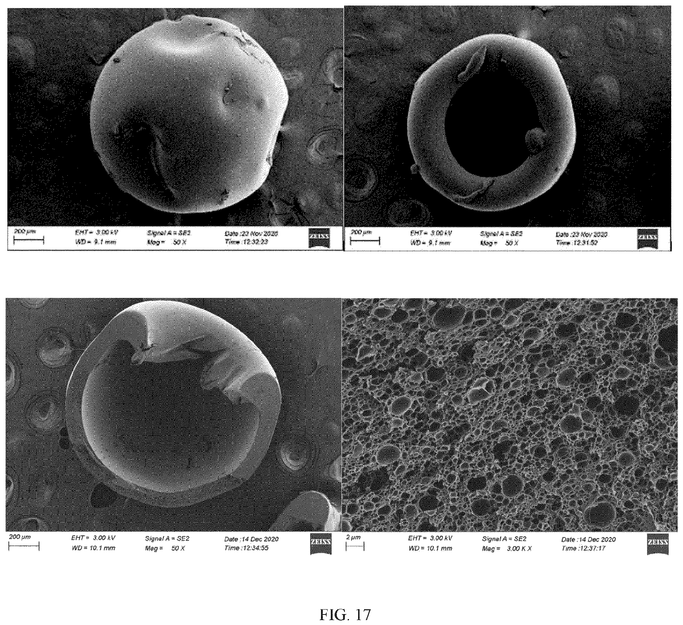

FIG. 17 shows SEM pictures of the hollow bowl-shaped microspheres prepared in Example 4; as shown in the drawing, the prepared microspheres are hemispherical, and have a cavity at interior and a circular opening structure at the round bottom thereof.

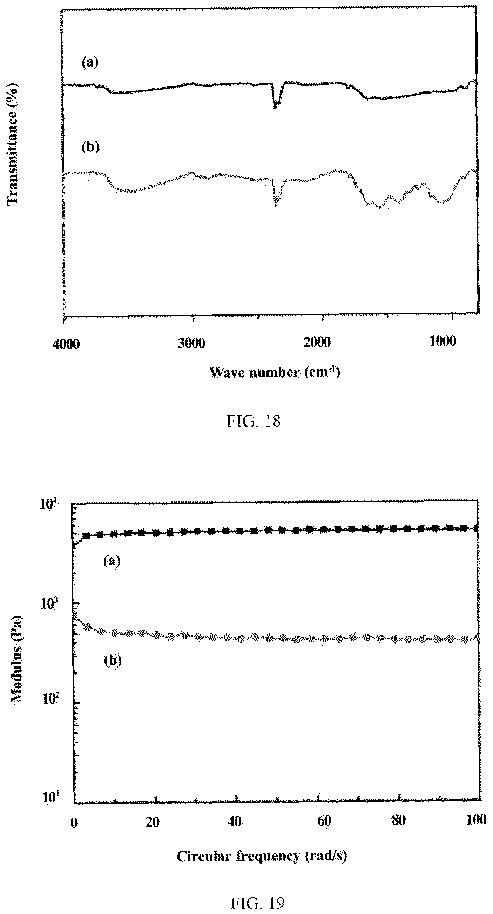

FIG. 18 shows Fourier infrared spectrum, wherein (a) is the Fourier infrared spectrum of the chitosan powder, and (b) is the Fourier infrared spectrum of the chitosan hollow bowl-shaped microspheres prepared in Example 4; the comparison of these two curves indicates successful internal crosslinking of microspheres.

FIG. 19 shows Rheological modulus curves of the hollow bowl-shaped microspheres prepared in Example 4, wherein (a) is the energy storage modulus curve and (b) is the loss modulus curve. As shown in the drawing, the energy storage modulus is higher than the loss modulus, indicating that the obtained microsphere is an elastic solid with excellent viscoelasticity and the ability of compressive deformation.

**Example 5: Preparation of carboxymethyl cellulose hollow bowl-shaped microspheres**

400 ul of 1,4-butanediol glycidyl ether was added into 20 ml of hydrochloric acid solution, of which pH is 5, and was subject to stirring for 30 minutes at 500 rpm; 0.4 g of carboxymethyl cellulose powder, of which the molecular weight is 700000, was added into the mixed solution, and was subject to stirring at room temperature for 48 hours at 1,500 rpm till sufficient dissolution of carboxymethyl cellulose, thereby obtaining carboxymethyl cellulose gel.

By using a syringe of 1 ml with a needle of 28 G having a beveled opening, the gel was slowly added dropwise into a beaker filled with 120 ml of isobutyl alcohol, and then was subject to stirring for 150 minutes at 1,500 rpm till the gel droplets in the solution changed from transparent to white solid microspheres.

The obtained solid microspheres were filtered out, and were dried in natural air for 48 hours at room temperature until the mass of the microspheres remained constant.

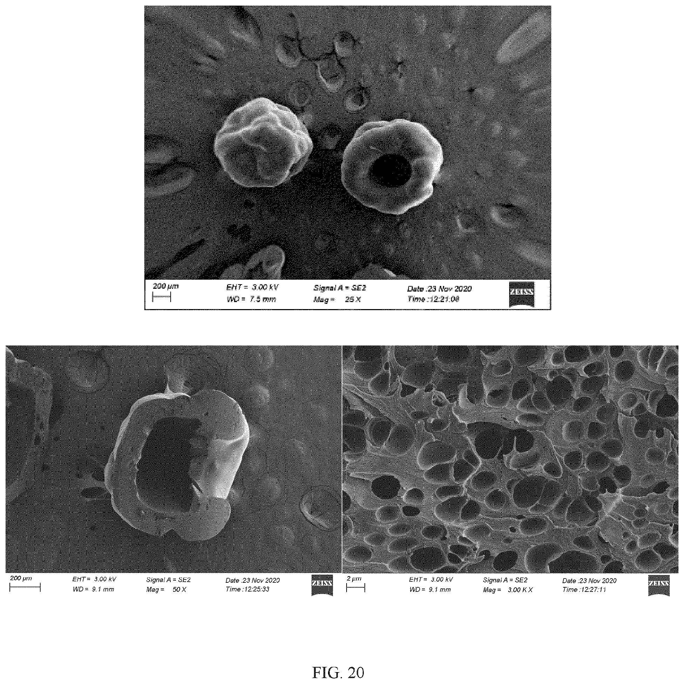

FIG. 20 shows SEM pictures of the hollow bowl-shaped microspheres prepared in Example 5; as shown in the drawing, the prepared microspheres are hemispherical, and each have a cavity at interior and a circular opening structure at the round bottom thereof.

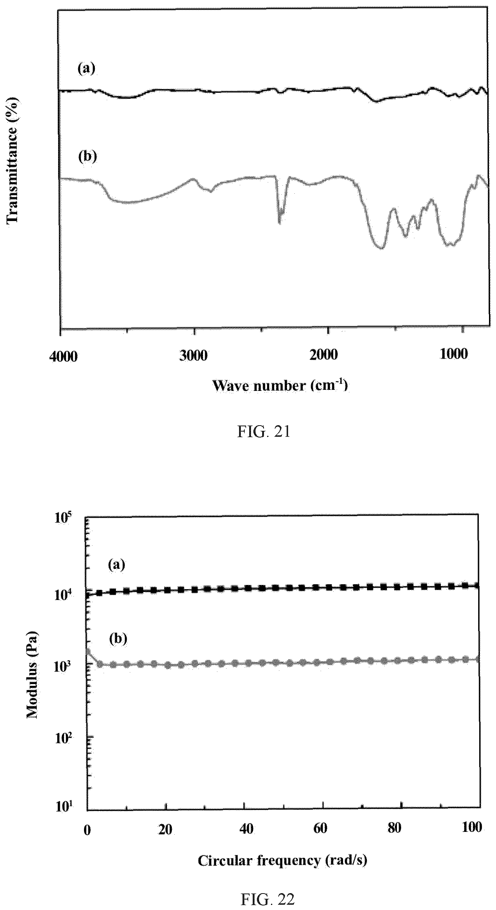

FIG. 21 shows Fourier infrared spectrum, wherein (a) is the Fourier infrared spectrum of the carboxymethyl cellulose powder, and (b) is the Fourier infrared spectrum of the carboxymethyl cellulose hollow bowl-shaped microspheres prepared in Example 5; the comparison of these two curves indicates successful internal crosslinking of microspheres.

FIG. 22 shows Rheological modulus curves of the hollow bowl-shaped microspheres prepared in Example 5, wherein (a) is the energy storage modulus curve and (b) is the loss modulus curve. As shown in the drawing, the energy storage modulus is higher than the loss modulus, indicating that the obtained microsphere is an elastic solid with excellent viscoelasticity and the ability of compressive deformation.

**Example 6: Preparation and characterization of sodium hyaluronate bowl-shaped hollow embolic microspheres loaded with iohexol**

1) Each of the four round-bottom flasks was added with 5 mL of hydrochloric acid solution, of which the pH is 5, then was respectively added with 50 $\mu$L of 1,4-butanediol glycidyl ether, and was subject to stirring for 20 minutes.

2) 50 mg, 0.2 g and 0.5 g of iohexol were weighed, and were respectively placed in three of the round-bottom flasks in Step 1), and then were subject to stirring.

3) 4 parts of sodium hyaluronate, each part being 0.1 g, were weighed, and then were respectively added to round-bottom flasks, of which 3 were added with iohexol and one was not, in Step 2), and then were subject to stirring.

4) The solution was slowly added to isopropyl alcohol dropwise, and then was subject to dehydration and cross-linking for 30 hours under high-speed stirring at 1,400 rpm, and microspheres were collected by means of centrifugation.

5) The obtained microspheres were placed in an oven of 25°C for 60 h until the mass of microspheres remained constant.

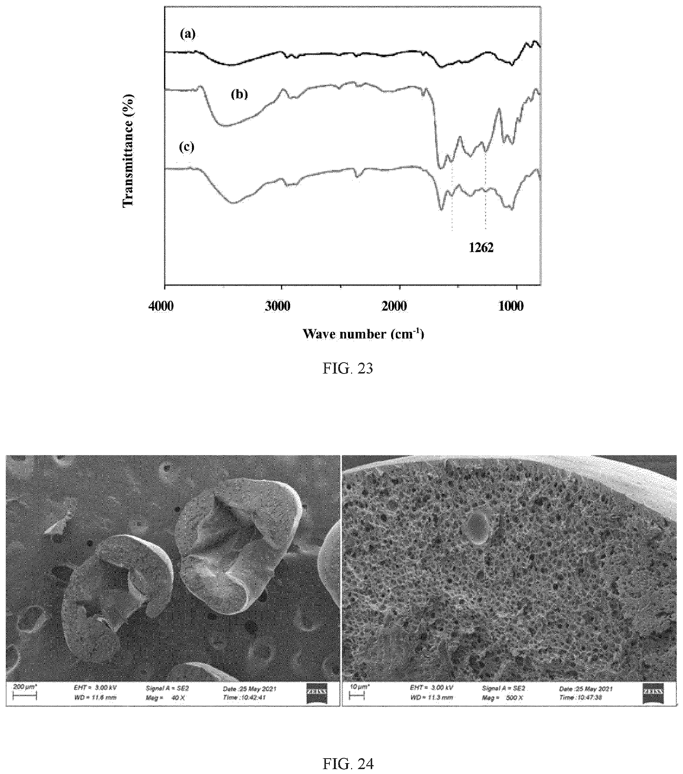

FIG. 23 shows Fourier infrared spectrum, wherein: (a) is the Fourier infrared spectrum of sodium hyaluronate bowl-shaped hollow embolic microspheres, (b) is the Fourier infrared spectrum of iohexol powder, and (c) is the Fourier infrared spectrum of sodium hyaluronate bowl-shaped hollow embolic microspheres loaded with 50 mg of iohexol. The comparison of adsorption peaks in the drawing indicates that the bowl-shaped hollow embolic microsphere in (c) has been loaded with iohexol.

FIG. 24 shows SEM pictures of the sodium hyaluronate bowl-shaped hollow microspheres, which were prepared in Example 6 and loaded with 50 mg of iohexol, cut in half, wherein the picture on the right of FIG. 3 is the one when the microsphere wall is enlarged by 500 times, and, as shown in the picture, the cross section of the microsphere has a porous structure.

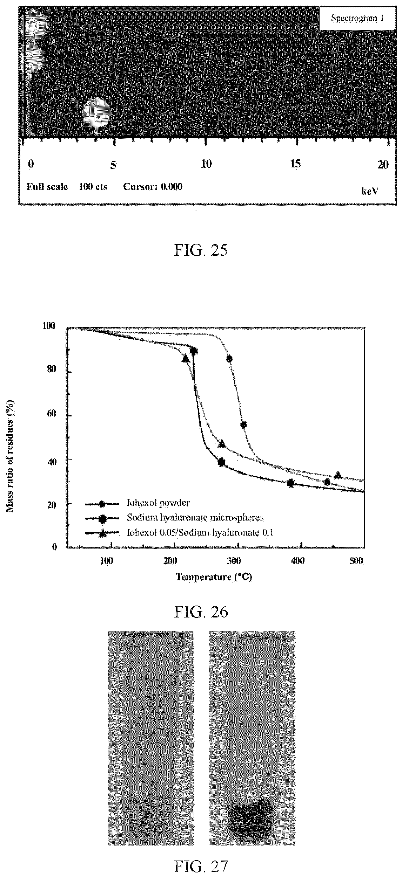

FIG. 25 shows energy spectrum picture of sodium hyaluronate bowl-shaped hollow microspheres that are prepared in Example 6 and loaded with 50 mg of iohexol, indicating successful loading of iohexol.

FIG. 26 shows thermogravimetric curves of sodium hyaluronate bowl-shaped hollow microspheres loaded with 50 mg of iohexol; as shown in the drawing, the residual amount of thermogravimetric curve of sodium hyaluronate bowl-shaped hollow microspheres loaded with 50 mg of iohexol is higher than that of simple sodium hyaluronate bowl-shaped microspheres, because the chemical composition of the microspheres changed after loaded with iohexol, and the thermal decomposition capacity of iohexol and that of sodium hyaluronate were different at the same temperature, which causes the residual amount of sodium hyaluronate bowl-shaped hollow microspheres loaded with iohexol increased, indicating successful loading of iohexol by the microspheres.

FIG. 27 shows X-CT images of microspheres that are prepared in Example 6 and loaded with different contents of iohexol, wherein: the left image shows the sodium hyaluronate bowl-shaped hollow embolic microspheres (not added with iohexol), and the right image shows the sodium hyaluronate bowl-shaped hollow embolic microspheres loaded with 50 mg of iohexol. Iohexol is a classical contrast medium for X-CT images, and, as shown by FIG. 6, microspheres loaded with 50 mg of iohexol have significant X-ray contrast effects.

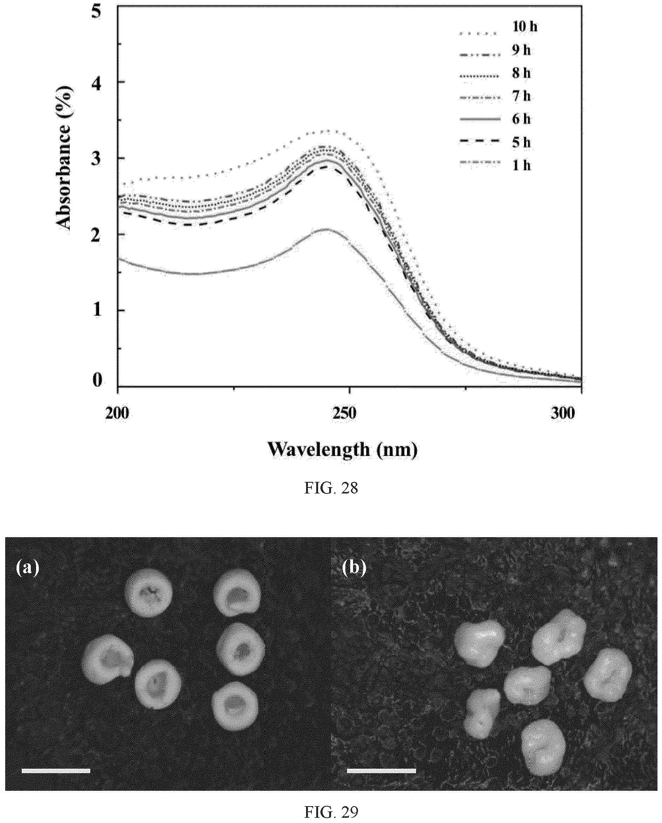

FIG. 28 shows drug release over time by the sodium hyaluronate bowl-shaped hollow microspheres that are prepared in Example 6 and loaded with 50 mg of iohexol, wherein the spot with a wavelength of 247 nm is the characteristic absorption peak of iohexol, and higher peak value indicates greater iohexol concentration. As shown by FIG. 7, the concentration of iohexol released into the aqueous solution increases with time.

FIG. 29 shows stereoscopic pictures of sodium hyaluronate bowl-shaped hollow embolic microspheres that are prepared in Example 6 and loaded with iohexol, wherein: (a) is the picture of sodium hyaluronate bowl-shaped hollow embolic microspheres loaded with 0.2 g of iohexol, with a mass ratio of sodium hyaluronate to iohexol during the preparation of microspheres being 1:2 (the theoretical drug loading at the moment was 66.7%), and a scale being 1 cm; and (b) is the picture of microspheres loaded with 0.5 g of iohexol, with a mass ratio of sodium hyaluronate to iohexol during the preparation of microspheres being 1:5 (the theoretical drug loading at the moment was 83.3%), and a scale being 1 cm; as shown in FIG. 29(b), the shape of microspheres changed irregularly, indicating that microspheres can no longer maintain the regular hollow bowl-shaped structure when the mass ratio of sodium hyaluronate to iohexol is 1:5, and showing that the maximum theoretical drug loading of iohexol for preparing the hollow bowl-shaped microspheres is between 66.7% and 83.3%.

**Example 7: Preparation and characterization of sodium hyaluronate bowl-shaped hollow embolic microspheres loaded with nano-tantalum powder**

1) 4 parts of sodium hyaluronate, each part being 0.1 g, were weighed, and then were respectively added to 4 round-bottom flasks.

2) Each of the four round-bottom flasks was added with 5 mL of hydrochloric acid solution, of which the pH is 5, then 4 parts of 1,4-butanediol glycidyl ether, each part being 5 mL, were respectively taken and dissolved in the 4 round-bottom flasks, and then stirring was performed for 20 minutes.

3) 50 mg, 1 g and 2 g of nano-tantalum powder were weighed, and were respectively placed in three of the round-bottom flasks in Step 2), and then were subject to stirring.

4) The solution in Step 3) was slowly added to n-amyl alcohol dropwise, and then was subject to dehydration and cross-linking for 24 hours under high-speed stirring, and microspheres were collected after still standing.

5) The obtained microspheres were placed in an oven of 25°C for 48 h until the mass of microspheres remained constant.

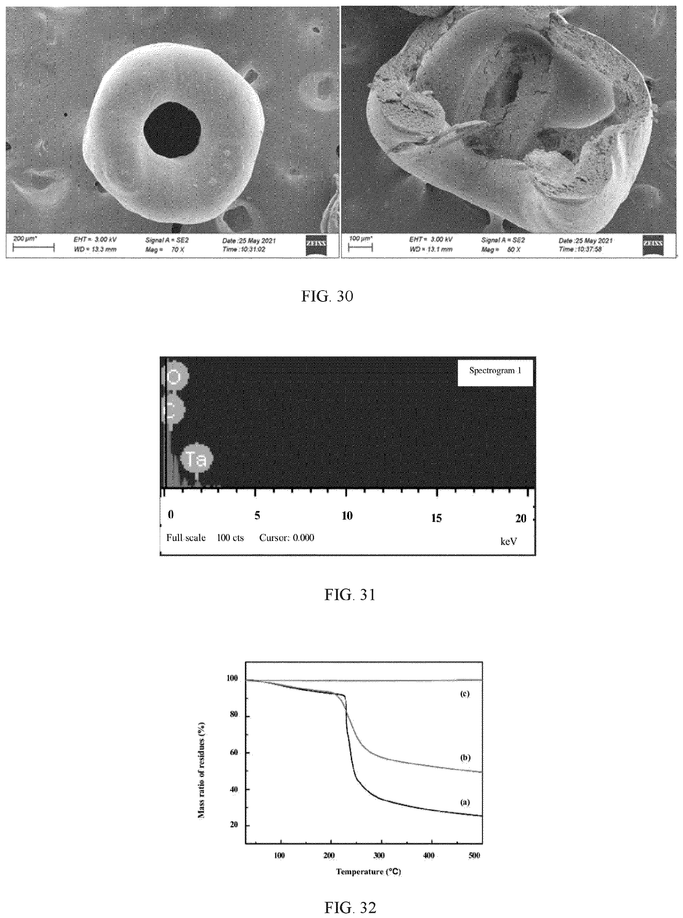

FIG. 30 shows SEM pictures of sodium hyaluronate bowl-shaped hollow embolic microspheres that are prepared in Example 7 and loaded with 50 mg of nano-tantalum powder, and, as shown by the drawing, the microspheres take a hollow bowl-shaped structure.

FIG. 31 shows an energy spectrum picture of a microsphere that is prepared in Example 7 and loaded with 50 mg of nano-tantalum powder; as shown by the drawing, tantalum element exists, proving loading of nano-tantalum powder into the microsphere.

FIG. 32 shows thermogravimetric curves, wherein: (a) is the thermogravimetric curve of pure sodium hyaluronate bowl-shaped hollow embolic microsphere without carbon powder, (b) is the thermogravimetric curve of sodium hyaluronate bowl-shaped hollow embolic microsphere loaded with 50 mg of nano-tantalum powder, and (c) is the thermogravimetric curve of pure nano-tantalum powder. The mass percentage of residual pure hyaluronic acid microspheres is 25.4%, the mass percentage of pure tantalum powder is 100%, and the mass percentage of residual hyaluronic acid microspheres loaded with 50 mg of nano-tantalum powder is 49.7%. According to the thermogravimetric curves, organic matters are prone to thermal decomposition and thus lose weight; microspheres added with nano-tantalum powder contains nano-tantalum powder, the mass of which remains almost unchanged in the process of burning, so the mass reduction of microspheres is caused by the mass decrease of sodium hyaluronate, as a result, the drug loading capacity and encapsulation efficiency of nano-tantalum powder can be calculated based on the weight difference between the microspheres loaded with nano-tantalum powder and those not. The calculation shows that the drug loading capacity of nano-tantalum powder is about 32%, and the drug encapsulation efficiency can be as high as 98%, indicating that the drug tantalum powder has almost no mass loss when tantalum powder is carried using the method of the present disclosure. This is determined by the preparation method of the microsphere, because the desiccant adsorbs and removes small-molecular water when liquid drops are gradually dropped into the desiccant, while the drug tantalum powder is retained in the polymer material due to its poor permeability in the polymer. It also makes clear that, as for the drug-loading microspheres prepared using the method of the present disclosure, the drug loading capacity of the microspheres is high, and a rather high drug encapsulation efficiency can be easily obtained.

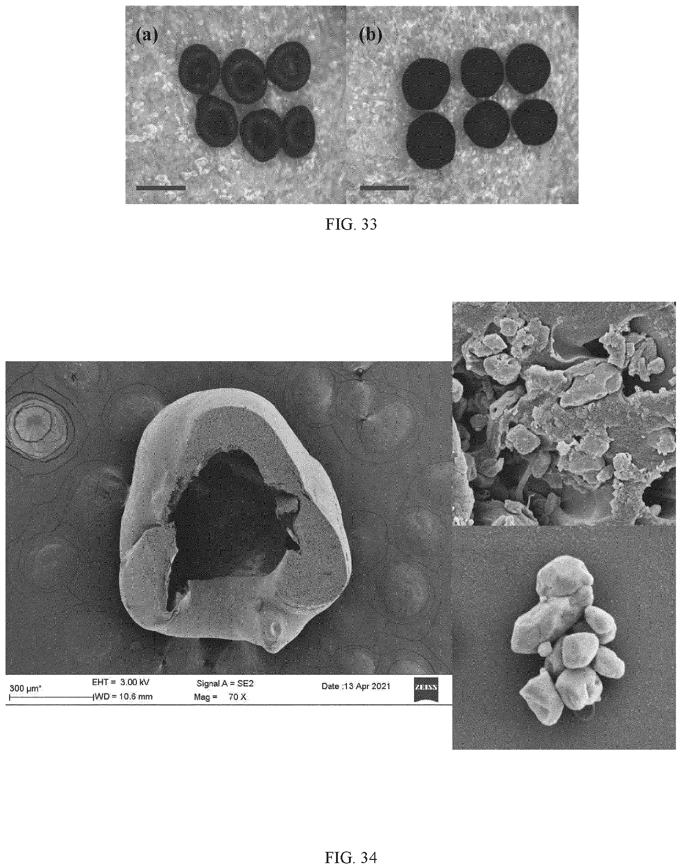

FIG. 33 shows stereoscopic pictures of sodium hyaluronate hollow embolic microspheres that are prepared in Example 7 and loaded with nano-tantalum powder, wherein (a) shows microsphere pictures where sodium hyaluronate is loaded with 1 g of nano-tantalum powder, with a mass ratio of sodium hyaluronate to nano-tantalum powder being 1:10 and a scale being 1 cm, the theoretical drug loading capacity at the moment is 90.9%, and the microspheres still have a complete bowl structure; (b) shows microsphere pictures of sodium hyaluronate loaded with 2 g of nano-tantalum powder, with a mass ratio of sodium hyaluronate to nano-tantalum powder being 1:20 and a scale being 1 cm. As shown in (b), in a case where the mass ratio of sodium hyaluronate to nano-tantalum powder is 1:20, the microsphere shape cannot be significantly affected by dehydration due to the small content of sodium hyaluronate, so the microsphere cannot form a hollow bowl-shaped structure.

**Example 8: Preparation and characterization of sodium hyaluronate loaded with barium sulfate bowl-shaped hollow embolic microspheres**

1) Each of five round-bottom flasks was added with 20 mL of hydrochloric acid solution, of which the pH is 5, then was respectively added with 200 μL of 1,4-butanediol glycidyl ether and was subject to stirring for 20 minutes.

2) 0.4, 0.2 g 0.1 and 0.02 g of barium sulfate were weighed, and were respectively placed in four of the round-bottom flasks in Step 1), and then were subject to stirring.

3) 5 parts of sodium hyaluronate, each part being 0.4 g, were weighed, and then were respectively added to round-bottom flasks, of which four were added with barium sulfate and one was not, in Step 2), and then were subject to stirring.

4) The solution was slowly added to isobutyl alcohol dropwise by using a bevel syringe with an inner diameter of 28 G, and then was subject to dehydration and cross-linking for 2 hours under high-speed stirring at 2,000 rpm, and microspheres were collected by means of centrifugation.

5) The obtained microspheres were placed in an oven of 25°C for 48 h until the mass of microspheres remained constant.

FIG. 34 shows SEM pictures of the cross-section of sodium hyaluronate hollow embolic microspheres that are prepared in Example 8 and loaded with 0.4 g of barium sulfate, from which it can be seen that the microsphere presents a hollow bowl-shaped structure; the upper right picture is an enlarged image of part of the cross section of the microsphere, and the lower right picture is the SEM image of pure barium sulfate; based on the comparison of the upper right picture and the lower right picture, it is clear that barium sulfate was encapsulated in the microsphere, and the addition of barium sulfate changed the microstructure of the wall of microsphere.

FIG. 35 shows the structure and element distribution diagrams of sodium hyaluronate hollow bowl-shaped microspheres that are prepared in Example 8 and loaded with 0.4 g of barium sulfate, wherein: (a) is the SEM picture of barium sulfate with a scale of 1 $\mu$m; (b) is the SEM picture of the top of the hollow bowl-shaped embolic microsphere with a scale of 200 $\mu$m; (c) is the SEM picture of the bottom of the hollow bowl-shaped embolic microsphere with a scale of 200 $\mu$m; (d) is the partially enlarged SEM picture of the bottom surface of the hollow bowl-shaped embolic microsphere with a scale of 10 $\mu$m; (e) is the SEM picture of the cross section, from the top to the bottom, of the hollow bowl-shaped embolic microsphere with a scale of 500 $\mu$m; (f) is the partially enlarged picture of the circle in (e) with a scale of 1 $\mu$m; (g) is the distribution diagram of various elements in the cross section of the hollow bowl-shaped embolic microsphere; (h) is the bar diagram of content proportions of elements including C, O, Na, Ba and S in the hollow bowl-shaped microsphere; (i1) is the distribution diagram of element C in sodium hyaluronate bowl-shaped microspheres loaded with 0.4 g of barium sulfate, (i2) is the distribution diagram of element O in sodium hyaluronate bowl-shaped microspheres loaded with 0.4 g of barium sulfate, (i3) is the distribution diagram of element Ba in sodium hyaluronate bowl-shaped microspheres loaded with 0.4 g of barium sulfate, and (i4) is the distribution diagram of element S in sodium hyaluronate bowl-shaped microspheres loaded with 0.4 g of barium sulfate. It can be seen from the drawings that the microsphere has a typical hollow bowl-shaped structure, it can be seen from (a) and (f) that barium sulfate is dispersed in the wall of the microsphere, and it can be seen from (i1) to (i4) that the elements C, O, Ba and S are evenly distributed in the microsphere, indicating that barium sulfate is evenly wrapped in the microsphere.

FIG. 36 shows Fourier infrared spectra, wherein: (a) is the Fourier infrared spectrum of barium sulfate, (b) is the Fourier infrared spectrum of sodium hyaluronate hollow bowl-shaped embolic microsphere, and (c) is the Fourier infrared spectrum of sodium hyaluronate hollow bowl-shaped embolic microsphere loaded with 0.4 g of barium sulfate; based on the comparison between the adsorption peaks of (a) and (b), additional adsorption bands from barium sulfate were observed in the FTIR spectrum (c) at 601 and 640 cm$^{-1}$, further proving successful loading of barium sulfate into the microspheres.

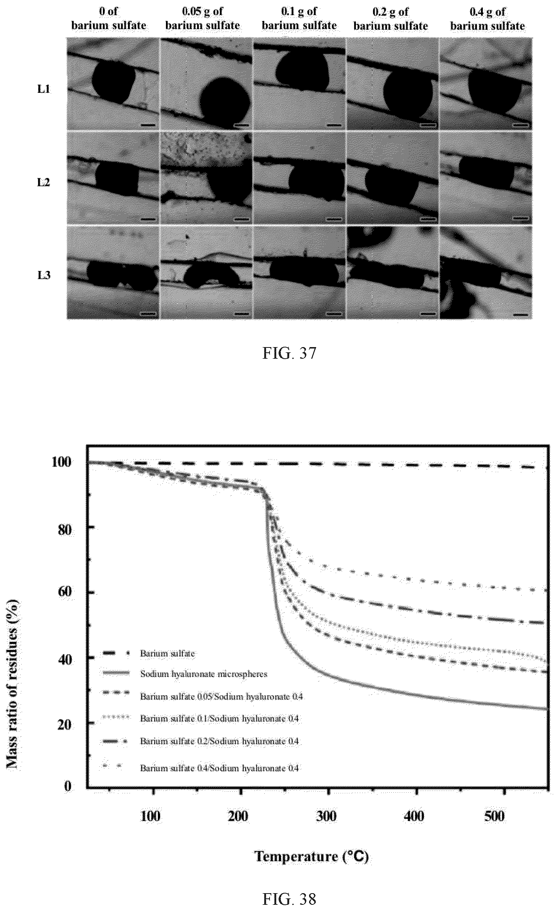

FIG. 37 shows test results of visual compression deformability of hollow bowl-shaped embolic microspheres, which are prepared in Example 8 and are respectively loaded with 0 g, 0.05 g, 0.1 g, 0.2 g and 0.4 g of barium sulfate, in channels (with a scale of 500 $\mu$m). As shown in the drawing, the three rows from L1 to L3 are respectively morphology changes of microspheres with different contents of barium sulfate in the channels when they move from a channel with a larger diameter to a channel with a smaller diameter. The hollow bowl-shaped embolic microspheres loaded with different amount of barium sulfate had a particle size of about 1,200 $\mu$m, all microspheres could successfully pass through channels with an inner diameter of 600 $\mu$m after pressure was applied to the channels, and none of the microspheres broke when the volume thereof was compressed to half of the original, indicating that the microspheres had good compression performance. All these five kinds of microspheres can move in a channel significantly smaller than their particle sizes, wherein microspheres loaded with 0 g and 0.05 g of barium sulfate will break when they move to a channel, of which the diameter is one third of the particle size of the microspheres, while microspheres loaded with 0.1 g, 0.2 g and 0.4 g of barium sulfate can still pass through the channel. It is clear that microspheres have intelligent deformability, and can pass through blood vessels with smaller diameters than that of the microspheres during transcatheter arterial embolization, thus achieving better embolization performance.

FIG. 38 shows thermogravimetric curves of the hollow bowl-shaped embolic microspheres that are prepared in Example 8 and loaded with barium sulfate. Based on these curves, it can be calculated that a mass percentage of residual hyaluronic acid is 25%; theoretical mass percentages of residual hollow bowl-shaped embolic microspheres loaded with 0.05 g, 0.1 g, 0.2 g and 0.4 g of barium sulfate are supposed to be 33%, 40%, 50% and 63%, while the actual mass percentages of residues are 36%, 39%, 51% and 60%, respectively; as a result, it can be calculated that the drug loading capacities of microspheres loaded with barium sulfate are respectively 14.7%, 18.7%, 34.6% and 47%, and the drug encapsulation efficiency respectively reaches 90% (according to the thermogravimetric curves, organic matters are prone to thermal decomposition and thus lose weight; microspheres added with barium sulfate contains barium sulfate, the mass of which remains almost unchanged in the process of burning, so the drug loading capacity of barium sulfate can be calculated based on the weight difference between the microspheres loaded with barium sulfate and those not).

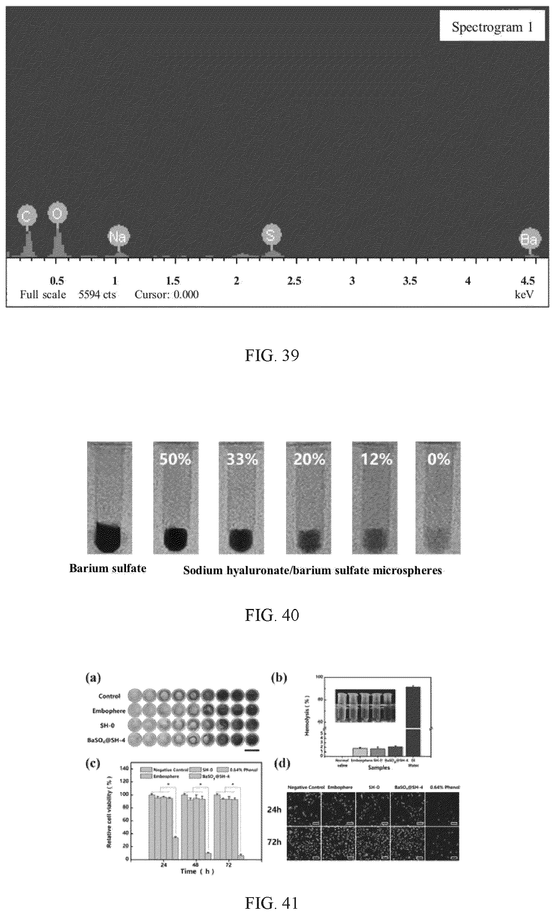

FIG. 39 shows the energy spectrum picture of sodium hyaluronate bowl-shaped hollow embolic microspheres that are prepared in Example 8 and loaded with 0.4 g of barium sulfate, and it can be seen that elements such as Ba and S exist in the microspheres, proving successful loading of barium sulfate.

FIG. 40 shows X-CT images of sodium hyaluronate bowl-shaped hollow embolic microspheres that are prepared in Example 8 and loaded with barium sulfate of different contents (content percentages of barium sulfate are calculated based on theoretical drug loading capacity); as shown by the drawings, the sodium hyaluronate bowl-shaped hollow embolic microspheres loaded with barium sulfate have excellent developing ability under X-rays, and the developing ability of microspheres becomes stronger and stronger with gradual increase of barium sulfate loading.

**Example 9: Biocompatibility experiment of sodium hyaluronate bowl-shaped hollow embolic microspheres loaded with barium sulfate**

1) Sodium hyaluronate bowl-shaped hollow embolic microspheres ($BaSO_4@SH$-4) that are prepared in Example 8 and loaded with 0.4 g of barium sulfate, and sodium hyaluronate bowl-shaped hollow embolic microspheres (SH-0) not loaded with barium sulfate were subjected to UV irradiation for 24 hours; then 200 mg of microspheres was weighed for each group and was mixed with 10 mL of DMEM (containing 10% FBS and 1% penicillin/streptomycin), and was incubated at 37°C for 24 hours.

2) A filter of 0.22 $\mu$m was used to deal with supernatant twice after infiltration of each microsphere, and then the supernatant was collected. HUVECs cells were incubated for 24 hours in a 96-well plate at a density of 5,000 cells/well, and original DMEM culture media were replaced with microsphere extracts (200 $\mu$L/well) of each component, and then culturing was performed for another 24 hours. Dimethyl sulfoxide (DMSO) of 5% was used as a positive control and untreated DMEM cell medium served as a negative control. 20 $\mu$L of CCK-8 solution was added to each well at 24, 48, and 72 hours and incubation was performed for 1 hour, and then absorbance at 450 nm was read with an enzyme-labeler.

3) In addition, a mixture, of which the density is 20 mg/mL, of microspheres loaded with each component and DMEM were co-cultured with bisected cells in a small culture dish for 24 hours and 72 hours. The medium and microspheres were removed, and then live/dead dyeing working solutions were respectively added to the culture dish and incubation was performed at 37°C for 30 minutes. After washed with PBS twice, the culture dish was observed and imaged by means of a confocal laser scanning microscope.

4) Sodium hyaluronate bowl-shaped hollow embolic microspheres ($BaSO_4@SH$-4) that are prepared in Example 8 and loaded with 0.4 g of barium sulfate, and sodium hyaluronate hollow bowl-shaped embolic microspheres (SH-0) were subject to ultraviolet radiation for 24 hours, and then 10 mg of microspheres of each component prepared in Example 3 was weighed respectively, was soaked in 1 mL of PBS and was incubated for 1 h at 37°C in a water bath. Incubated microspheres were transferred into a 96-well plate, and were respectively added with 100 $\mu$L of citric acid rabbit whole blood, with PBS and Embosphere® microspheres as control groups.

5) After that, 100 $\mu$L of 0.1M $CaCl_2$ solution was added to each well to trigger the blood clotting reaction, and the well plate was shaken horizontally during the process. At different time intervals, 200 $\mu$L of 0.109 M sodium citrate solution were respectively added to stop the clotting. Ultra-pure water was used for washing for three times. Finally, pictures were taken for recording.

6) Sodium hyaluronate bowl-shaped hollow embolic microspheres ($BaSO_4@SH$-4) that are prepared in Example 8 and loaded with 0.4 g of barium sulfate, and sodium hyaluronate hollow bowl-shaped embolic microspheres (SH-0) were subject to ultraviolet radiation for 24 hours, and then 10 mg of microspheres of each component prepared in Example 3 was weighed respectively, was soaked in 1 mL of PBS solution and was incubated for 1 h at 37°C. Then, 500 $\mu$L of citric acid rabbit whole blood was added into 24.5 mL of PBS solution for 50-fold dilution. 1 mL of diluted blood was added to solution of microspheres of each component, and the solution was subject to further incubation at 37°C for 4 hours and then to centrifugation for 10 minutes at 10,000 rpm. After that, 100 $\mu$L of supernatant was collected from each group. PBS solution and ultrapure water component were respectively used as negative and positive control groups. The absorbance of hemolytic sample was recorded at 545 nm by an enzyme-labeler, and the calculation is as follows:

$$\text{Hemolysis (\%)}=(ODE-ODN)/ODP\times100\%$$

wherein: ODE is the absorbance of the sample at 545 nm, ODN is the absorbance of the negative control, and ODP is the absorbance of the positive control.

FIG. 41 shows evaluation result images of in vitro biocompatibility of sodium hyaluronate bowl-shaped hollow embolic microspheres ($BaSO_4@SH$-4) that are prepared in Example 8 and loaded with 0.4 g of barium sulfate, and sodium hyaluronate hollow bowl-shaped embolic microspheres (SH-0). (a) is the clotting experiment-plasma re-calcification time determination result (clotting performances of each component from left to right are respectively 1 to 9 minutes); it can be seen that clotting gradually started from the 3rd minute, and became more and more significant over time; compared with Embosphere®, SH-0 component microspheres and $BaSO_4@SH$-4 component microspheres were not significantly different in clotting, indicating that the clotting time of BaSO$_4$@SH-4 microspheres produces clotting effects similar to those of products currently on the market; besides, at each time interval tested, BaSO$_4$@SH-4 microspheres had a clot no smaller than that of PBS control groups, indicating that BaSO$_4$@SH-4 microspheres did not cause increased clotting reaction in case of direct contact with blood; prior to the two components including SH-0 and BaSO$_4$@SH-4, there was no significant difference in clotting either, meaning that BaSO$_4$ did not affect microsphere thrombosis due to its relatively stable in vivo chemical properties and would not pose potential biological toxicity. (b) is the hemolytic experiment results (hemolytic experiment was conducted for implanted devices, and the relative concentration of hemoglobin was determined through determination of the OD value of the solution, thus reflecting the hemolysis related properties of prepared embolic microspheres), wherein the hollow bowl-shaped microspheres prepared in this experiment belonged to interventional devices in contact with blood, and the hemolysis ratio thereof was much lower than 5%, which satisfies the international requirements. (c) is the diagram showing results of HUVEC cell proliferation assay measured by means of CCK-8 method (relative cell survival rates of SH-0, Embosphere® and BaSO$_4$@SH-4 components all reached 90%, indicating that all groups maintained rather good cell viability and low toxicity. However, cell viability obtained using phenol solution (positive control) of 0.64% was rather low, decreasing from 34.0% to 6.2% during incubation. These results showed that the infiltrates of BaSO$_4$@SH microspheres showed no obvious cytotoxicity). (d) is a diagram showing growth of HUVEC cells observed by means of live and dead dyeing; compared with a negative control group, distribution and morphology of live cells (dyed green) showed normal growth on the first and third day; almost no dead cell was observed in the experimental group and the negative control group, but obvious cell death appeared in the positive control group; in addition, the number of cells in the experimental group is similar to that of the negative control group, which was consistent with CCK-8 results.

**Example 10: Kidney embolization experiment of a beagle by means of sodium hyaluronate bowl-shaped hollow embolic microspheres loaded with barium sulfate**

1) A beagle kidney model was adopted to evaluate in vivo embolization effects of BaSO$_4$@SH-4 microspheres prepared in Example 8, wherein an embolic agent was injected into one kidney of the beagle, and then the effect of BaSO$_4$@SH microspheres, as an embolic agent, was evaluated based on comparison of the renal necrosis on the embolized side and that on the unembolized side.

2) A healthy beagle weighed 8 to 10 kg was acclimated for at least 1 week under standard feed requirements, then was anesthetized by intramuscular injection with 30 mg/kg of pentobarbital sodium of 2%, and was subject to a surgery so as to expose the right femoral artery. The surgery was performed by a special surgeon, and observation was made under X-ray during the surgery (Digital subtraction angiography, DSA, Infinix-IINFX-8000C, Toshiba).

3) To obtain a renal angiography, Omnipaque (a contrast agent) was injected into the beagle's kidney, and then 300 mg of BaSO$_4$@SH-4 (100 mg was 350-500 μm, 100 mg was 475-675 μm, and 100 mg was 700-900 μm, respectively) was injected into a target renal artery after mixed with 5 mL of Omnipaque. Subsequently, repeated angiography was performed to confirm successful blocking of blood flowing to the kidney.

FIG. 42 shows diagrams of beagle kidney embolization by means of sodium hyaluronate bowl-shaped hollow microspheres that are prepared in Example 8 and loaded with 0.4 g of barium sulfate, wherein: (a) shows the image of a normal kidney under DSA without embolization after injection of iohexol solution, from which a whole reticular structure of the right kidney can be seen. (b) and (c) clearly show the process of the X-ray opaque BaSO$_4$@SH-4 microsphere moving from blood vessels to the kidney with the flow of fluid. (d) is the DSA image of the kidney after embolization by microspheres, from which no blood flow in the kidney was visible, indicating successful embolization of the kidney by the microspheres. (e) and (f) are X-CT images of the kidney after embolization, showing that microspheres loaded with 0.4 g of barium sulfate have a certain development ability under CT after embolization.

FIG. 43 is the diagram showing pathological examination of tissue of a beagle kidney embolized by means of sodium hyaluronate bowl-shaped hollow embolic microspheres that are prepared in Example 8 and loaded with 0.4 g of barium sulfate, wherein: (a) shows images of an embolized kidney after 1 and 4 weeks after surgery and that of a normal kidney, with a scale of 1 cm; (b) shows cross-sectional pictures of (a), with a scale of 1 cm; the unembolized control kidney was the other kidney at 1 week of embolization, of which both the outer surface and the cross section were smooth, indicating no ischemic necrosis caused by non-target embolization. As for the short-term embolized kidney (1 week), local ischemic necrosis (white hyperplasia) occurred at the edge of the kidney, and slight atrophy at the edge could be seen in the sectional view, indicating successful occlusion of renal artery by BaSO$_4$@SH-4 microspheres with no significant changes in the size of the kidney. Moreover, due to the effective blocking of blood vessels by BaSO$_4$@SH-4 microspheres, the volume of the kidney decreased noticeably after 4 weeks of embolization, no complete structure of the kidney could be seen in the sectional view, and the kidney had been completely atrophied. Yellowish-green purulent fluid could be seen in the sectional view, indicating that prolonged embolization had caused obvious immune rejection. These data further proved that the target kidney had been effectively embolized for up to 4 weeks by $BaSO_4$@SH-4. (c) shows H&E dyeing at the first and fourth week of a normal kidney and that of an embolized kidney, with a scale of 100 $\mu$m; and (d) shows enlarged images of (c), with a scale of 200 $\mu$m. As can be seen from the H&E-dyed sections, control groups were visible, the cells were evenly distributed, the structure was clear, the boundary between the cortex and medulla was clearly visible, and the shape of glomerulus was normal and intact. In the section of a kidney embolized for one week, the number of lobular cells increased in the glomeruli, indicating abnormal proliferation of cells. But the whole structure was not obviously different from that of the control kidney, and the glomerular shape was normal, indicating that the kidney could still function to certain extent at this time. However, in the section of a kidney embolized for four weeks, the structure of the kidney was abnormal, a large number of inflammatory cells had accumulated in the glomeruli, and glomerular necrosis accompanied by a large amount of calcification were caused by cortical ischemia. The pink area in the middle indicated protein deposition and significant fibrosis, and the kidney at this moment had lost all its functions.

FIG. 44 shows histopathological examination of tissue of a beagle kidney embolized by means of sodium hyaluronate bowl-shaped hollow embolic microspheres ($BaSO_4$@SH-4) that are prepared in Example 8 and loaded with 0.4 g of barium sulfate, wherein: (a) shows the CT image of an unembolized kidney collected at the fourth week, with a scale of 2 cm; (b) shows H&E dyeing of renal artery in (a), with a scale of 100 $\mu$m; (c) shows an enlarged picture of (b), with a scale of 200 $\mu$m; (d) shows the CT image of the embolized kidney at the fourth week, with a scale of 2 cm; (e) shows H&E dyeing of renal artery in (d), with a scale of 100 $\mu$m; and (f) is an enlarged image of (e), with a scale of 200 $\mu$m; CT images of the embolized kidney showed a clear and complete vascular network structure, indicating that the excellent radiopaque $BaSO_4$@SH-4 microspheres stayed stably inside the blood vessels. Besides, no significant white spots were found in the unembolized kidney, suggesting that the microspheres did not migrate from the target kidney to the non-target kidney on the opposite side. CT images further proved that $BaSO_4$@SH-4 microspheres were capable of penetrating into the distal renal artery. As shown by the H&E-dyed section, representative H&E images at the renal artery showed that blood vessels of renal artery on the embolized side had been completely filled with endothelial cells, while the control group showed obvious blank areas at the same position, indicating that continuous (4 weeks) embolization enabled the cells to grow along microspheres, as a result, the arterial blood vessels had been completely covered with fibrotic tissue, through which blood flow could not pass, and the embolization effect would last forever. In addition, the embolized kidney did not significantly affect the other one, and there was no non-target embolization.

## Example 11: Preparation and characterization of hyaluronic acid bowl-shaped hollow embolic microspheres loaded with HAp-$Fe_3O_4$ nanoparticles

Each of two round-bottom flasks was added with 5 mL of deionized water, 2 parts of hyaluronic acid, each part being 0.1 g, were weighed, and then 2 parts of 1,4-butanediol glycidyl ether, each part being 50 $\mu$L, were respectively taken and dissolved in the two round-bottom flasks, and then stirring was performed for 20 minutes. 50 mg of HAp-$Fe_3O_4$ nanoparticles was weighed, and was placed in one of the round-bottom flasks, and then was subject to stirring. Then the mixed solution was slowly added to isobutyl alcohol dropwise, and then were subject to dehydration and cross-linking for 30 hours under high-speed stirring at 1,400 rpm, and microspheres were collected after still standing. The obtained microspheres were placed in an oven of 40°C for 12 h until the mass of microspheres remained constant.

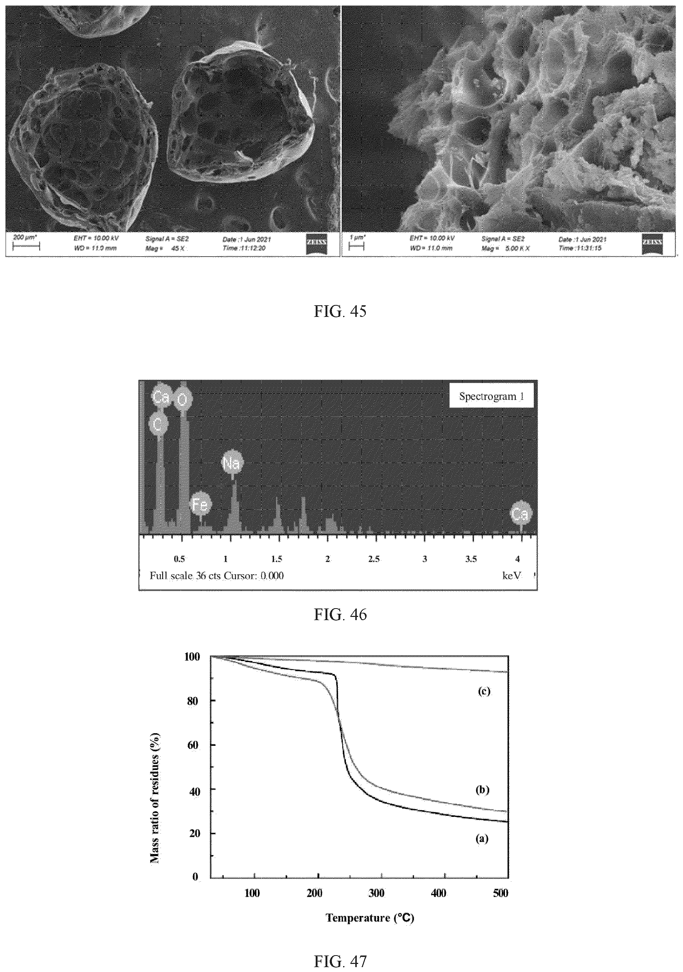

FIG. 45 shows SEM pictures of hollow bowl-shaped embolic microspheres that are prepared in Example 11 and loaded with 50 mg of HAp-$Fe_3O_4$ nanoparticles, and it can be seen from the drawing that the microspheres are in a hollow bowl shape and have pore structures in the cross section thereof.

FIG. 46 shows energy spectrum pictures of hyaluronic acid bowl-shaped hollow embolic microspheres prepared in Example 11, and it can be seen that Fe, Ca and other elements are contained in the microspheres, which proves successful loading of HAp-$Fe_3O_4$ nanoparticles in the microspheres.

FIG. 47 shows thermogravimetric curves of hyaluronic acid bowl-shaped hollow embolic microspheres prepared in Example 11, wherein: (a) represents hyaluronic acid bowl-shaped hollow embolic microspheres; (b) represents hyaluronic acid bowl-shaped hollow embolic microspheres loaded with 50 mg of HAp-$Fe_3O_4$ nanoparticles; and (c) represents HAp-$Fe_3O_4$ nanoparticles, from which it can be seen that the maximum loading capacity thereof is 5% (according to the thermogravimetric curves, organic matters are prone to thermal decomposition when they are burned and thus lose weight; microspheres loaded with HAp-$Fe_3O_4$ nanoparticles contains HAp-$Fe_3O_4$ nanoparticles, the weight of which remains almost unchanged in the process of burning, so the loading capacity of HAp-$Fe_3O_4$ nanoparticles can be calculated based on the weight difference between the microspheres loaded with HAp-$Fe_3O_4$ nanoparticles and those not).

**Example 12: Preparation and characterization of sodium hyaluronate bowl-shaped hollow embolic microspheres loaded with Mn₃O₄ nanoparticles**

1) Each of two round-bottom flasks was added with 5 mL of hydrochloric acid solution, of which the pH is 5, then was respectively added with 50 $\mu$L of 1,4-butanediol glycidyl ether and was subject to stirring for 20 minutes.
2) 50 mg of $Mn_3O_4$ nanoparticles was weighed, and was placed in one of the round-bottom flasks in Step 1), and was subject to stirring.
3) 2 parts of sodium hyaluronate, each part being 0.1 g, were weighed, and then were respectively added to round-bottom flasks in Step 2), of which one was added with $Mn_3O_4$ nanoparticles and the other was not, and then were subject to stirring.
4) The solution was slowly added to isobutyl alcohol dropwise, and then was subject to dehydration and cross-linking for 24 hours under high-speed stirring, and microspheres were collected by means of centrifugation.
5) The obtained microspheres were respectively placed in an oven of 25°C for 48 h until the mass of microspheres remained constant.

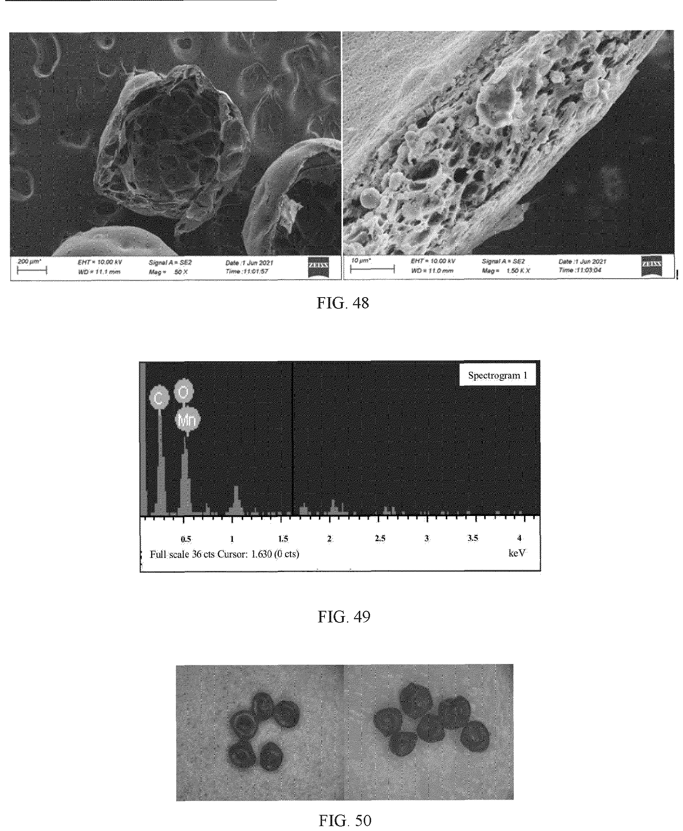

FIG. 48 shows SEM pictures of sodium hyaluronate bowl-shaped hollow microspheres that are prepared in Example 12 and loaded with 50 mg of $Mn_3O_4$ nanoparticles, and it can be seen from the drawing that the microspheres are in a hollow shape and have pore structures in the cross section thereof.
FIG. 49 shows SEM energy spectrum picture of sodium hyaluronate bowl-shaped hollow embolic microspheres that are prepared in Example 12 and loaded with 50 mg of $Mn_3O_4$ nanoparticles, wherein Mn, O and other elements can be seen in the drawing, which proves successful loading of $Mn_3O_4$ nanoparticles.

**Example 13: Preparation and characterization of sodium hyaluronate bowl-shaped hollow embolic microspheres loaded with doxorubicin hydrochloride**

1) Each of three round-bottom flasks was added with 5 mL of hydrochloric acid solution, of which the pH is 5, then was respectively added with 50 $\mu$L of 1,4-butanediol glycidyl ether and was subject to stirring for 20 minutes.
2) 5 mg and 8 mg of doxorubicin hydrochloride were weighed, and were placed in two of the round-bottom flasks in Step 1), and were subject to stirring.
3) 3 parts of sodium hyaluronate, each part being 0.1 g, were weighed, and then were respectively added to the round-bottom flasks in Step 2), of which two was added with doxorubicin hydrochloride and the other was not, and then were subject to stirring.
4) The solution was slowly added to isobutyl alcohol dropwise, and then was subject to dehydration and cross-linking for 24 hours under high-speed stirring, and microspheres were collected by means of centrifugation.
5) The obtained microspheres were respectively placed in an oven of 25°C for 48 h until the mass of microspheres remained constant.

FIG. 50 shows stereoscopic pictures of sodium hyaluronate hollow embolic microspheres that are prepared in Example 13 and loaded with doxorubicin hydrochloride, wherein (a) is the microsphere picture with sodium hyaluronate loaded with 5 mg of doxorubicin hydrochloride, the mass ratio of sodium hyaluronate to doxorubicin hydrochloride being 20: 1; and (b) is the microsphere picture with sodium hyaluronate loaded with 8 mg of doxorubicin hydrochloride, the mass ratio of sodium hyaluronate to doxorubicin hydrochloride being 25:2. As shown by the drawings, in a case where the mass ratio is 25:2, since sodium hyaluronate has a negative charge and doxorubicin hydrochloride has a positive charge, the large charge polarity difference may easily result in powder agglomeration, as a result of which uniform gel cannot be formed.

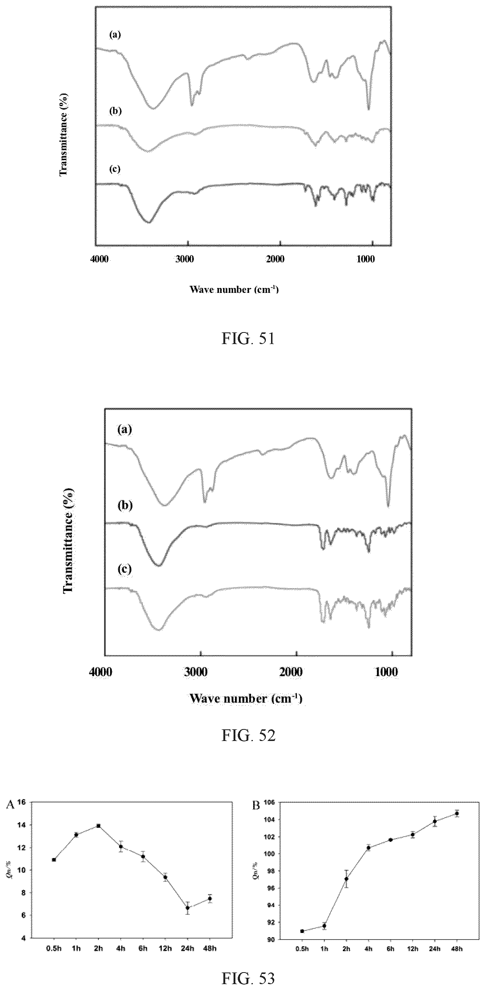

FIG. 51 shows the Fourier infrared spectrum, wherein: (a) represents sodium hyaluronate bowl-shaped hollow embolic microspheres, (b) represents doxorubicin hydrochloride powder, and (c) represents sodium hyaluronate bowl-shaped hollow embolic microspheres loaded with 5 mg of doxorubicin hydrochloride powder; and the comparison of adsorption peaks indicates successful loading of doxorubicin hydrochloride in microspheres.

**Example 14: Preparation and characterization of sodium hyaluronate bowl-shaped hollow embolic microspheres loaded with paclitaxel**

1) Each of two round-bottom flasks was added with 5 mL of hydrochloric acid solution, of which the pH is 5, then was respectively added with 50 μL of 1,4-butanediol glycidyl ether and was subject to stirring for 20 minutes.

2) 5 mg of paclitaxel was weighed, and was placed in one of the round-bottom flasks in Step 1), and was subject to stirring.

3) 2 parts of sodium hyaluronate, each part being 0.1 g, were weighed, and then were respectively added to round-bottom flasks in Step 2), of which one was added with paclitaxel and the other was not, and then were subject to stirring.

4) The solution was slowly added to isobutyl alcohol dropwise, and then was subject to dehydration and cross-linking for 24 hours under high-speed stirring, and microspheres were collected by means of centrifugation.

5) The obtained microspheres were respectively placed in an oven of 25°C for 48 h until the mass of microspheres remained constant.

FIG. 52 shows the Fourier infrared spectrum, wherein: (a) represents sodium hyaluronate bowl-shaped hollow embolic microspheres, (b) represents paclitaxel powder, and (c) represents sodium hyaluronate bowl-shaped hollow embolic microspheres loaded with 5 mg of paclitaxel powder; and the comparison of adsorption peaks in (a) and (b) indicates successful loading of d paclitaxel therein.

**Example 15: Preparation and characterization of sodium hyaluronate hollow bowl-shaped embolic microspheres loaded with Rhodamine B and doxorubicin hydrochloride**

1) Each of three round-bottom flasks was added with 20 mL of hydrochloric acid solution, of which the pH is 5, then was respectively added with 200 μL of 1,4-butanediol glycidyl ether and was subject to stirring for 20 minutes.

2) 12 mg of Rhodamine B and doxorubicin hydrochloride were respectively weighed, and were placed in two of the round-bottom flasks in Step 1), and were subject to stirring.

3) 3 parts of sodium hyaluronate, each part being 0.4 g, were weighed, and then were respectively added to the round-bottom flasks in Step 2), of which one was added with Rhodamine B, one was added with doxorubicin hydrochloride and another one was not added with Rhodamine B or doxorubicin hydrochloride, and then were subject to stirring.

4) The solution was slowly added to isobutyl alcohol dropwise, and then was subject to dehydration and cross-linking for 24 hours under high-speed stirring, and microspheres were collected by means of centrifugation.

5) The obtained microspheres were respectively placed in an oven of 25°C for 48 h until the mass of microspheres remained constant.

6) 0.1 g of prepared sodium hyaluronate hollow bowl-shaped embolic microspheres loaded with 12 mg of Rhodamine B and 0.1 g of those loaded with doxorubicin hydrochloride were respectively taken, and were equally placed into six EP tubes of 15 mL, into which 10 mL of PBS buffer solution was added (pH=7.4); then the EP tubes were fixed in a constant temperature incubation oscillator with the temperature set at 37°C. 1 mL of sample to be measured was taken respectively at 0.5 h, 1 h, 2 h, 4 h, 6 h, 12 h, 24 h and 48 h, and the same volume of PBS buffer was added for refilling.

7) Ultraviolet light adsorption of the solution was measured by ultramicro-spectrophotometer, concentrations of Rhodamine B and doxorubicin hydrochloride were read via standard curves at corresponding time intervals, and the release rate was calculated.

FIG. 53 shows in vitro drug release of sodium hyaluronate bowl-shaped hollow microspheres that are prepared in Example 15 and respectively loaded with 12 mg of Rhodamine B and doxorubicin hydrochloride, wherein the release rate of sodium hyaluronate microspheres was $90.1\pm0.2\%$ after loaded with Rhodamine B for 30 minutes. After replacement of fresh PBS, the cumulative release exceeded 100%, and the possible cause was re-dissociation of agglomerated Rhodamine B. As shown by the cumulative release curves of doxorubicin hydrochloride, burst release first occurred when a large number of naked carboxyl groups existing in the raw material sodium hyaluronate was effectively combined with doxorubicin hydrochloride, wherein the maximum release rate during 48 h was $13.93\pm0.1\%$; after that, the drug in the solution recombined with hyaluronic acid, the concentration slowly decreased, and dissociation and binding finally reached equilibrium; without renewing the solvent, the concentration of doxorubicin in the solvent tended to be stable, with a release rate of $7.47\pm0.2\%$ at 48 h.

**Example 16**

50 ul of 1,4-butanediol glycyl ether was added into 20 ml of hydrochloric acid solution, of which pH is 5, and was subject to stirring for 30 minutes at 500 rpm; 0.4 g of sodium hyaluronate powder, of which the molecular weight is 1 to 1.5 million daltons, was added into the mixed solution, and was subject to stirring, at room temperature for 24 hours at 500 rpm,

till sufficient dissolution of the sodium hyaluronate powder, thereby obtaining sodium hyaluronate gel; the gel was transferred to a bio-3D printer, and hollow bowl-shaped embolic microspheres were obtained by printing according to 3D printing program.

**Claims**

1. A hollow embolic microsphere, wherein: the hollow embolic microsphere has a spheroidal or hemispheroidal shape, an internal cavity and an opening structure, and the material of the hollow embolic microsphere is biocompatible polymer material with viscoelasticity; preferably, the hollow embolic microsphere is in a bowl shape, a spherical shape, a hemispherical shape, an ellipsoidal shape, or a short cylinder shape with a spherical head; preferably, the particle diameter range of the hollow embolic microsphere is 50 to 2,000 $\mu$m, preferably 200 to 2,000 $\mu$m, and more preferably 200 to 1,200 $\mu$m; preferably, the hollow embolic microspheres may be a combination of multiple groups of microspheres with different particle size distributions, for example, one or a combination of two or more groups of microspheres with particle size of 50 to 120 $\mu$m, microspheres with particle size of 300 to 500 $\mu$m, microspheres with particle size of 500 to 700 $\mu$m, microspheres with particle size of 700 to 900 $\mu$m, and microspheres with particle size of 900 to 1,200 $\mu$m; for another example, one or a combination of two or more groups of microspheres with particle size of 200 to 300 $\mu$m, 300 to 500 $\mu$m, 500 to 700 $\mu$m, 700 to 900 $\mu$m, and 900 to 1,200 $\mu$m; preferably, the inner diameter of the opening structure is 1% to 95% of the particle size of the hollow embolic microsphere; and more preferably, the inner diameter of the opening structure is 10% to 80% of the particle size of the hollow embolic microsphere.

2. The hollow embolic microsphere according to claim 1, wherein the opening of the hollow embolic microsphere has a bulge towards the center of the opening structure or the center of the cavity.

3. The hollow embolic microsphere according to claim 1 or 2, wherein: if the direction from the top to the opening at the bottom is the height direction, and the direction perpendicular to the height direction is the width direction, then the ratio of width to height of the hollow embolic microsphere is 1:10 to 10:1, preferably 1:3 to 3:1, and more preferably 1:2 to 2:1; the ratio of the inner diameter to the outer diameter of the opening at the opening structure of the hollow embolic microsphere is 1:50 to 9:10, preferably 1:5 to 4:5, and more preferably 1:4 to 1:2; and the ratio of the wall thickness of the hollow embolic microsphere to the microsphere particle size is 1:10 to 1:1, preferably 1:5 to 4:5, and more preferably 1:4 to 2:3.

4. The hollow embolic microsphere according to any one of claims 1 to 3, wherein: the material of the hollow embolic microsphere is one or more polymers selected from polyvinyl alcohols, polyesters, proteins and sugars; preferably, it is one or more polymers selected from proteins and sugars, more preferably, one or more polymers selected from sugars, and, more preferably, one or more polymers selected from gelatin, alginic acid, chitosan, carboxymethyl cellulose, and hyaluronic acid; more preferably, it is one or more polymers selected from alginic acid, chitosan, carboxymethyl cellulose, hyaluronic acid, and their salts (preferably sodium salt, potassium salt); more preferably, it is one or more polymers selected from sodium alginate, potassium alginate, sodium carboxymethyl cellulose, potassium carboxymethyl cellulose, sodium hyaluronate, potassium hyaluronate, chitosan, carboxymethyl cellulose, and hyaluronic acid; preferably, the relative molecular mass of the hyaluronic acid or hyaluronate used is 10,000 to 20,000,000 daltons, with preferable molecular weight of 100,000 to 8,000,000 daltons, and more preferable molecular weight of 500,000 to 3,000,000 daltons; preferably, the molecular weight of alginate or its salt is 10,000 to 500,000 daltons, and more preferably 100,000 to 200,000 daltons; preferably, the molecular weight of carboxymethyl cellulose or its salt is 10,000 to 1,000,000 daltons, and more preferably 300,000 to 800,000 daltons; preferably, the molecular weight of chitosan is 10,000 to 800,000 daltons, and more preferably 50,000 to 300,000 daltons; preferably, the polymer material is a crosslinked polymer material; preferably, the crosslinking agent used in the crosslinked polymer material is selected from one or a combination of several selected from divinyl sulfone, carbodiimide, dihydrazide adipate, 1,4-butanediol glycidyl ether and glutaraldehyde; more preferably, the crosslinking agent is dihydrazide adipate, 1,4-butanediol glycidyl ether or a combination thereof with other crosslinking agents, and, more preferably is 1,4-butanediol glycidyl ether or a combination thereof with other crosslinking agents; preferably, the mass ratio of the crosslinking agent to the polymer material is 0.0001:1 to 10:1, more preferably 0.01:1 to 2:1, more preferably 0.01:1 to 1:1, more preferably 0.1:1 to 2:1, more preferably 0.1:1 to 1:1.

5. A method for preparing the hollow embolic microsphere according to any one of claims 1 to 4, wherein the microsphere is obtained by means of 3D printing.

6. A method for preparing the hollow embolic microsphere according to any one of claims 1 to 4, including steps of:

   1) dissolving the polymer material in water, an acidic aqueous solution or an alkaline aqueous solution, and stirring for full dissolution; optionally, adding a crosslinking agent and performing sufficient mixing;
   2) adding dropwise the mixed liquid prepared in Step 1) to a dehydrant that is being stirred, and microspheres are formed after dehydration; and
   3) collecting the prepared microspheres and drying them;

   wherein: there is no restriction on the order for adding the polymer material and the crosslinking agent in Step 1).

7. The method according to claim 6, wherein: the concentration of the polymer material prepared in Step 1) is 0.5% to 5%, and preferably 1% to 3.5%; in a case where the polymer material in Step 1) is one or a combination of several of hyaluronic acid, alginic acid and carboxymethyl cellulose, it is preferable that the polymer material is dissolved in water; in a case where the polymer material in Step 1) is a polymer material containing chitosan, it is preferable that the polymer material is dissolved in an acidic aqueous solution, and the pH of the aqueous solution is preferably 1 to 6.9, more preferably 4 to 6.8; preferably, the acidic aqueous solution is hydrochloric acid solution, sulfuric acid solution, nitric acid solution, or glacial acetic acid solution; preferably, the alkaline aqueous solution is sodium hydroxide, or potassium hydroxide aqueous solution.

8. The method according to claim 6 or 7, wherein: the crosslinking agent in Step 1) is one or a combination of several selected from divinyl sulfone, carbodiimide, dihydrazide adipate, 1,4-butanediol glycidyl ether, and glutaraldehyde; preferably, the crosslinking agent is dihydrazide adipate, 1,4-butanediol glycidyl ether or a combination thereof with other crosslinking agents, and, more preferably, 1,4-butanediol glycidyl ether or a combination thereof with other crosslinking agents; preferably, the mass ratio of the crosslinking agent to the polymer material is 0.0001:1 to 10:1, more preferably 0.01:1 to 2:1, more preferably 0.01:1 to 1:1, more preferably 0.1:1 to 2:1, and more preferably 0.1:1 to 1:1.

9. The method according to any one of claims 6 to 8, wherein a syringe or microfluidic device is adopted to control the size of the droplets added in the dropwise adding of the mixed liquid in Step 2), so as to obtain desired particle size or a combination of particle size distributions.

10. The method according to any one of claims 6 to 9, wherein: the dehydrant in Step 2) is alcohol solvent, preferably alcohol solvent with 3 to 5 carbon atoms, preferably one or several combined solvents with a total content of 95% or more and selected from isopropyl alcohol, isobutyl alcohol, n-amyl alcohol, and isoamyl alcohol, and more preferably one or several combined solvents selected from anhydrous isopropyl alcohol, anhydrous isobutyl alcohol, anhydrous n-amyl alcohol, and anhydrous isoamyl alcohol.

11. The method according to any one of claims 6 to 10, wherein: the volume ratio of the dehydrant in Step 2) to the polymer solution is 4:1 or more, preferably 10:1 or more, and more preferably 20:1 or more.

12. The method according to any one of claims 6 to 11, wherein: the stirring speed in Step 2) is 100 to 2,000 rpm, and preferably 800 to 1,500 rpm; the dehydration stirring time is 0.1 hours or more, and preferably 0.4 to 4 hours; preferably, 20-50°C oven drying or natural wind evaporation drying or low temperature freeze-drying is adopted in Step 3).

13. The hollow embolic microsphere according to any one of claims 1 to 4, wherein the hollow embolic microspheres are drug-loading hollow embolic microspheres; preferably, the loaded drug is one or a combination of several selected from nanoparticles, micron particles, small molecule drugs, chemotherapeutic drugs, fluorescent markers, macromolecular drugs, peptides, plasmids, and viral drugs; preferably, the micron particles or nanoparticles may be listed, for example, as one or a combination of several selected from: metal micron particles, metal nanoparticles, magnetic micron particles, magnetic nanoparticles, inorganic non-metallic micron particles, inorganic non-metallic nanoparticles, polymer micron particles, polymer nanoparticles, nano-micelles, and nano-liposomes; preferably one or a combination of several selected from nano-hydroxyapatite, nano-tantalum powder, nano-calcium phosphate, nano-manganese phosphate, nano-manganese dioxide, nano-iron oxide, nano-barium sulfate, and iohexol; the micron particles can preferably be listed, for example, as one or a combination of several selected from barium sulfate, calcium carbonate microspheres, calcium phosphate microspheres, and aluminum oxide microspheres; the chemotherapeutic drugs, small molecule drugs and macromolecular drugs can be listed, for example, as one a combination of several selected from doxorubicin hydrochloride, paclitaxel, cisplatin, fluorouracil, interferon, aptamers, Sorafenib, Osimertinib, Afatinib, Ceritinib, Palbocillin, Everolimus, Regorafenib, imatinib, Axitinib, Pazopanib, Ibrutinib, Vermotinib, Sonidegib, hyaluronidase, nivolumab, Pertuzumab, Pembrolizumab, Bevacizumab, and antibody conjugated drugs; the fluorescent markers may be listed, for example, as FITC and/or Rhodamine B; the peptides drug may be listed, for example, as one or a combination of several selected from leuprorelin, goserelin, triptorelin, and Nafarelin; the plasmid drugs can be listed, for example, as one or a combination of several selected from pEGFP plasmid, pCMVp-NEO-BAN plasmid, pSV2 plasmid, CMV4 plasmid, pUC18 plasmid, pUC19 plasmid, and pcDNA3.4 plasmid; it is optional that the plasmid drugs carry therapeutic genes, and, preferably, the therapeutic genes include p53 gene, thymidine kinase gene, cytidine deaminase gene, granulo-macrophage colony-stimulating factor gene (GM-CSF), CRISP/Cas9 gene system, anticancer embryo gene, anti-angiogenesis gene, anti-transferrin gene or gene analogitics thereof; and the viral drugs may be listed, for example, as one or a combination of several selected from oncolytic adenoviruses with or without inserted therapeutic genes, oncolytic gland-associated viruses, oncolytic lentiviruses, oncolytic vaccinia viruses, oncolytic herpes simplex viruses, measles viruses, and virus-like microspheres.

14. A method for preparing the hollow embolic microsphere according to claim 13, including adding the drugs in Step 1) of the method according to any one of claims 6 to 12, wherein: there is no restriction on the order for adding the polymer material, the crosslinking agent and the drug;
alternatively, in the method according to any one of claims 6 to 12, after dry microspheres are obtained in Step 3), the microspheres are placed in the drug solution to adsorb the drug; preferably, the microspheres are dried after adsorbing the drug.

15. The method according to claim 14, wherein: in Step 1), the mass ratio of the added drug to the polymer material is 0.000001:1 to 10:1, and the mass of the nanoparticles and micron particles added to the polymer material solution per gram of the polymer material is preferably 0.001 to 10 g/g, more preferably 0.01 to 5 g/g; the mass of protein drugs, small molecule drugs, chemotherapeutic drugs and nano drugs added to per gram of polymer materials is at mg level, such as 1 to 2,000 mg/g; the mass of plasmid drugs loaded in each gram of polymer material is at $\mu$g level, such as 1 to 3,000 $\mu$g/g;
alternatively, after the microspheres are dried and obtained in Step 3), the microspheres are placed in the drug solution to adsorb drugs, and the mass of protein drugs, small molecule drugs, chemotherapeutic drugs and nano drugs adsorbed by per gram of the polymer material is at mg level, such as 1 to 2,000 mg/g; the mass of plasmid drugs adsorbed by each gram of polymer material is at $\mu$g level, such as 1 to 3,000 $\mu$g/g; preferably, the dose of viral drugs adsorbed by per cubic centimeter volume of hollow embolic microspheres is 10^6 to 10^12 Pfu/ml (Pfu: plaque-forming units), and preferably 10^7 to 10^10 Pfu/ml, such as oncolytic adenovirus of 10^6 to 10^8 Pfu/ml, oncolytic herpes simplex virus of 10^6 to 10^8 Pfu/ml, and poxvirus of 10^7 to 10^9 Pfu/ml.

16. A hollow embolic microsphere prepared using the preparation method according to any one of claims 5 to 12 and 14 to 15.

17. A pharmaceutical composition or a medical device, comprising the hollow embolic microsphere according to any one of claims 1 to 4, 13 and 16.

18. Use of the hollow embolic microsphere according to any one of claims 1 to 4, 13 and 16 or the pharmaceutical composition according to claim 17 in the preparation of drug or medical device for transcatheter arterial embolization.

19. Use of the hollow embolic microsphere according to any one of claims 1 to 4, 13 and 16 or the pharmaceutical composition according to claim 17 in the preparation of drugs for tumors, hemorrhagic lesions, arteriovenous malformations, prostatic hyperplasia (also referred to as PAE), scar pregnancy, chronic osteoarthritis, uterine adenomyosis, and hypersplenism; preferably, the tumors include malignant tumors, or uterine fibroids (also referred to as UAE).

FIG. 1

FIG. 2

FIG. 3

FIG. 4

FIG. 5

FIG. 6

FIG. 7

FIG. 8

FIG. 9

FIG. 10

FIG. 11

FIG. 12

FIG. 13

FIG. 14

FIG. 15

FIG. 16

FIG. 17

FIG. 18

FIG. 19

FIG. 20

FIG. 21

FIG. 22

FIG. 23

FIG. 24

FIG. 25

FIG. 26

FIG. 27

FIG. 28

FIG. 29

FIG. 30

FIG. 31

FIG. 32

FIG. 33

FIG. 34

FIG. 35

FIG. 36

FIG. 37

FIG. 38

FIG. 39

Barium sulfate   Sodium hyaluronate/barium sulfate microspheres

FIG. 40

FIG. 41

FIG. 42

FIG. 43

FIG. 44

FIG. 45

FIG. 46

FIG. 47

FIG. 48

FIG. 49

FIG. 50

FIG. 51

FIG. 52

FIG. 53

<div style="text-align:center">**INTERNATIONAL SEARCH REPORT**</div>

| International application No. |
| --- |
| **PCT/CN2023/091079** |

**A. CLASSIFICATION OF SUBJECT MATTER**

A61L24/08(2006.01)i; A61L24/00(2006.01)i; A61L31/04(2006.01)i; A61L31/14(2006.01)i; A61L31/16(2006.01)i; A61L31/18(2006.01)i; A61K47/69(2017.01)i; A61K47/36(2006.01)i; A61K45/00(2006.01)i

According to International Patent Classification (IPC) or to both national classification and IPC

**B. FIELDS SEARCHED**

Minimum documentation searched (classification system followed by classification symbols)

IPC:A61L A61F A61K

Documentation searched other than minimum documentation to the extent that such documents are included in the fields searched

Electronic data base consulted during the international search (name of data base and, where practicable, search terms used)

CJFD, CNABS, CNTXT, ENTXT, ENTXTC, VEN, VCN, CNKI, 万方 WANFANG, 超星读秀学术 CHAOXINGDUXIU SCHOLAR, Elsevier Science, STN on the web: 3D打印, 北京坦路来专利代理有限公司, 打印, 交联, 开口, 颗粒, 孔祥东, 粒子, 罗丹丹, 马矢徒, 腔, 球粒, 沈杨, 栓塞, 孙志超, 脱水, 碗, 微粒, 微球, 微珠, 易子晗, 赵瑞波, 浙江理工大学, 中空, 祖柏尔, cavity, dehydrate, dehydration, embolic, embolism, embolize, grain, granu, granule, hollow, microballoon, open, particle, powder, cross link+, dehydrat+, microsphere+, microbead+, cross-link+, crosslink+, bowl

**C. DOCUMENTS CONSIDERED TO BE RELEVANT**

| Category* | Citation of document, with indication, where appropriate, of the relevant passages | Relevant to claim No. |
| --- | --- | --- |
| PX | CN 115054724 A (ZHEJIANG SCI-TECH UNIVERSITY) 16 September 2022 (2022-09-16) claims 1-19 | 1-19 |
| PX | YI, Zihan et al. "The sodium hyaluronate microspheres fabricated by solution drying for transcatheter arterial embolization" *Journal of Materials Chemistry B*, Vol. 10, No. 21, 28 April 2022 (2022-04-28), pages 4105-4114 ISSN: 2050-7518, abstract, page 4106, right column, paragraph 2 to page 4112, right column, paragraph 2 | 1-19 |
| X | CN 112023115 A (MU YONGXU) 04 December 2020 (2020-12-04) description, paragraphs [0019]-[0058], and figures 1-7 | 1-4, 13 |
| Y | CN 112023115 A (MU YONGXU) 04 December 2020 (2020-12-04) description, paragraphs [0019]-[0058], and figures 1-7 | 5-12, 14-19 |

☑ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **08 August 2023** | **16 August 2023** |

| Name and mailing address of the ISA/CN | Authorized officer |
| --- | --- |
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

International application No.

**PCT/CN2023/091079**

## C. DOCUMENTS CONSIDERED TO BE RELEVANT

| Category* | Citation of document, with indication, where appropriate, of the relevant passages | Relevant to claim No. |
|---|---|---|
| Y | CN 112569878 A (SUZHOU HENGRUI HONGYUAN MEDICAL TECHNOLOGY CO., LTD.) 30 March 2021 (2021-03-30)<br>description, paragraphs [0013]-[0051] | 5, 16-19 |
| Y | BABO, Pedro S et al. "Production and characterization of hyaluronic acid microparticles for the controlled delivery of growth factors using a spray/dehydration method"<br>*Journal of Biomaterials Applications,* Vol. 31, No. 5, 19 September 2016 (2016-09-19), pages 1-15<br>ISSN: 0885-3282,<br>page 3 paragraph 2, page 7 paragraph 2, and figures 1-3 | 6-12, 14-19 |
| Y | CN 112957517 A (SUZHOU MICROSCALE TECHNOLOGY CO., LTD.) 15 June 2021 (2021-06-15)<br>description, paragraphs [0021]-[0055] | 6-12, 14-19 |
| A | CN 107185029 A (NANJING UNIVERSITY) 22 September 2017 (2017-09-22)<br>entire document | 1-19 |
| A | US 2018265402 A1 (DALHOUSIE UNIVERSITY) 20 September 2018 (2018-09-20)<br>entire document | 1-19 |

Form PCT/ISA/210 (second sheet) (July 2022)

**INTERNATIONAL SEARCH REPORT**
Information on patent family members

International application No.

**PCT/CN2023/091079**

| Patent document cited in search report | | | Publication date (day/month/year) | Patent family member(s) | | | Publication date (day/month/year) |
|---|---|---|---|---|---|---|---|
| CN | 115054724 | A | 16 September 2022 | None | | | |
| CN | 112023115 | A | 04 December 2020 | None | | | |
| CN | 112569878 | A | 30 March 2021 | CN | 112569878 | B | 28 September 2021 |
| CN | 112957517 | A | 15 June 2021 | CN | 112957517 | B | 29 November 2022 |
| CN | 107185029 | A | 22 September 2017 | None | | | |
| US | 2018265402 | A1 | 20 September 2018 | WO | 2017054076 | A1 | 06 April 2017 |
| | | | | EP | 3356305 | A1 | 08 August 2018 |
| | | | | EP | 3356305 | B1 | 04 November 2020 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **SHETH R A** ; **SHARJEEL S** ; **SAVITRI K**. EndovascμLar embolization by transcatheter delivery of particles: past, present, and future.. *Journal of Functional Biomaterials*, 2017, vol. 8 (2), 12-20
- **POURSAID A** ; **JENSEN M M** ; **HUO E**. Polymeric materials for embolic and chemoembolic applications [J].. *Journal of Controlled Release*, 2016, vol. 240, 414-433

- **WENG L** ; **LE H C** ; **TALAIE R**. Bioresorbable hydrogel microspheres for transcatheter embolization: preparation and in vitro evaluation[J].. *Journal of VascμLar&Interventional Radiology*, 2011, vol. 22 (10), 1464-1470